# EUROPEAN PATENT APPLICATION

(11) **EP 4 813 439 A1**
(43) Date of publication of application: **30.09.2026**
(21) Application number: 24893232.9
(22) Date of filing: 05.11.2024
(51) Int. Cl.: A61M 60/178, A61M 60/865

(54) **BLOOD PUMP**

(30) Priority: 21.11.2023 CN 202311568122; 29.12.2023 CN 202311861519
(71) Applicant: Shenzhen Core Medical Technology Co., Ltd., Shenzhen, Guangdong 518000 (CN)
(72) Inventor: XIE, Duanqing, Shenzhen, Guangdong 518000 (CN); DENG, Dazhao, Shenzhen, Guangdong 518000 (CN); HUANG, Jiaming, Shenzhen, Guangdong 518000 (CN); YU, Shunzhou, Shenzhen, Guangdong 518000 (CN)
(74) Representative: Petraz, Gilberto Luigi
(86) International application number: PCT/CN2024/129963
(87) International publication number: WO 2025/108074

(57) **Abstract**

A blood pump (10) is provided, which includes a pump body (11). The pump body (11) includes a pump casing (100), a driving unit (200), and an atraumatic ring (300). A proximal end of the pump casing (100) is provided with a liquid inlet (101). A distal end of the pump casing (100) is open to define the distal end of the pump casing (100) as a liquid outlet (102), and a central axis of the pump casing (100) passes through the liquid outlet (102). The driving unit (200) is fixedly connected to the proximal end of the pump casing (100), and is configured to drive blood to flow from the liquid inlet (101) to the liquid outlet (102). The atraumatic ring (300) is disposed around a peripheral edge of the liquid outlet (102). A radial thickness of the atraumatic ring (300) is greater than a wall thickness of the pump casing (100). The atraumatic ring (300) is provided with an annular wall (310) extending in a circumferential direction of the atraumatic ring (300), and a center of a generating circle of a torus in which the annular wall (310) is located is positioned inside the atraumatic ring (300).

## Description

This application claims priority to Chinese Patent Application No. 202311568122.6, filed November 21, 2023, and entitled "BLOOD PUMP", and claims priority to Chinese Patent Application No. 202311861519.4, filed December 29, 2023, and entitled "BLOOD PUMP", the entire disclosures of both of which are incorporated herein by reference.

### TECHNICAL FIELD

The present disclosure relates to the field of medical equipment technology, and in particular, to a blood pump.

### BACKGROUND

In the related art, there is a blood pump capable of assisting the right ventricle in pumping blood. The delivery path for implanting such a blood pump generally extends from the inferior vena cava or superior vena cava, and sequentially passes through the right atrium, the tricuspid valve, the right ventricle, and the pulmonary valve to reach the pulmonary artery. This right ventricular delivery path is characterized by its short path, numerous bends, and complex internal tissue structures. Consequently, during implantation, the blood pump often needs to make multiple turns to navigate these bends of the delivery path. However, a distal portion of a conventional blood pump is highly prone to colliding or rubbing against internal tissues when navigating such bends along the delivery path, thereby causing damage to the internal tissues.

The above information disclosed in the background art of the disclosure is only for understanding the background of the concept of the disclosure and may not constitute related art.

### SUMMARY

Based on this, the disclosure provides a blood pump, which can reduce collision or friction between the blood pump and internal tissues during implantation of the blood pump, so as to avoid damaging the internal tissues.

The disclosure provides a blood pump, and the blood pump includes a pump body. The pump body includes a pump casing, a driving unit, and an atraumatic ring. A proximal end of the pump casing is provided with a liquid inlet. A distal end of the pump casing is open to define the distal end of the pump casing as a liquid outlet, and a central axis of the pump casing passes through the liquid outlet. The driving unit is fixedly connected to the proximal end of the pump casing, and is configured to drive blood to flow from the liquid inlet to the liquid outlet. The atraumatic ring is disposed around a peripheral edge of the liquid outlet. A radial thickness of the atraumatic ring is greater than a wall thickness of the pump casing. The atraumatic ring is provided with an annular wall extending in a circumferential direction of the atraumatic ring, and a center of a generating circle of a torus in which the annular wall is located is positioned inside the atraumatic ring.

The disclosure further provides a blood pump, and the blood pump includes a pump body. The pump body includes a pump casing, an atraumatic ring, and a driving unit. The pump casing includes a main body section having a liquid inlet and a variable diameter section connected to the main body section. The atraumatic ring is disposed at a distal end of the variable diameter section. An inner circumference of the atraumatic ring and an inner circumference of the variable diameter section together define a liquid outlet. A distal end of the atraumatic ring is provided with an atraumatic wall, the atraumatic wall extends in a circumferential direction of the atraumatic ring, and a radial width of the atraumatic wall in a radial direction of the pump casing is greater than a wall thickness of the pump casing. The driving unit includes an impeller disposed inside the pump casing. The impeller faces the liquid outlet, a direction from the impeller to the liquid outlet is defined as a first direction, and a diameter of the liquid outlet gradually increases in the first direction, such that the liquid outlet has a flared shape.

Details of one or more embodiments of the disclosure are set forth in the accompanying drawings and illustration below. Other features, objectives, and advantages of the disclosure become clear from this specification, the accompanying drawings, and the claims.

### BRIEF DESCRIPTION OF THE DRAWINGS

In order to more clearly illustrate the technical solutions in the embodiments of the disclosure, the following will briefly introduce the drawings required in the illustration of the embodiments or the related art. Obviously, the drawings in the following are only some embodiments of the disclosure. For those of ordinary skill in the art, other drawings can be obtained based on these drawings without creative effort.
FIG. 1 is a schematic structural view of a blood pump provided in an embodiment of the disclosure.
FIG. 2 is a front view of the blood pump illustrated in FIG. 1.
FIG. 3 is a cross-sectional view of the structure illustrated in FIG. 2, taken along line I-I.
FIG. 4 is a schematic view of the structure illustrated in FIG. 3 with some dimensions labeled.
FIG. 5 is a schematic exploded structural view of the blood pump illustrated in FIG. 1.
FIG. 6 is a schematic view illustrating blood flow when a pump body of the blood pump of the disclosure is positioned in a pulmonary artery.
FIG. 7 is a schematic view illustrating an extension tube and an atraumatic ring of the blood pump illustrated in FIG. 1 integrally connected.
FIG. 8 is a longitudinal cross-sectional view of the structure illustrated in FIG. 7.
FIG. 9 is an enlarged view of a portion P1 of the structure illustrated in FIG. 8.
FIG. 10 is a schematic view of the structure illustrated in FIG. 9 with dimensions labeled.
FIG. 11 is a schematic structural view of a blood pump provided in another embodiment of the disclosure.
FIG. 12 is a schematic view of an internal structure of the blood pump illustrated in FIG. 11.
FIG. 13 is a schematic structural view of a blood pump provided in yet another embodiment of the disclosure.
FIG. 14 is a front view of the blood pump illustrated in FIG. 13.
FIG. 15 is a cross-sectional view of the structure illustrated in FIG. 14, taken along line I-I.
FIG. 16 is a schematic view of the structure illustrated in FIG. 15 with some dimensions labeled.
FIG. 17 is an enlarged view of a portion P2 of the structure illustrated in FIG. 16.
FIG. 18 is a top view of the blood pump illustrated in FIG. 13.
FIG. 19 is an exploded structural schematic view of the blood pump illustrated in FIG. 13.
FIG. 20 is a schematic view showing an extension tube and an atraumatic ring of the blood pump illustrated in FIG. 19 integrally connected.
FIG. 21 is a longitudinal cross-sectional view of the structure illustrated in FIG. 20.
FIG. 22 is an enlarged view of a portion P3 of the structure illustrated in FIG. 21.
FIG. 23 is a schematic view illustrating included angles between tangent lines of a portion of the structure illustrated in FIG. 22.
FIG. 24 is a schematic view showing a pump body of the blood pump of the disclosure being clamped and fixed by the pulmonary valve.
FIG. 25 is a schematic view showing the blood pump of the disclosure being implanted into the pulmonary artery from a first blood pathway.
FIG. 26 is a schematic view showing the blood pump of the disclosure being implanted into the pulmonary artery from a second blood pathway.
FIG. 27 is a schematic view showing a distal end of a blood pump in the related art in contact with an inner tissue wall.
FIG. 28 is a schematic view showing a distal end of a blood pump of the disclosure in contact with an inner tissue wall.

### DETAILED DESCRIPTION

In order to make the above-mentioned objects, features, and advantages of the disclosure more obvious and comprehensive, a detailed illustration of specific embodiments of the disclosure will be given below in conjunction with the accompanying drawings. In the following illustration, many specific details are provided to facilitate full understanding of the disclosure. However, the disclosure can be implemented in many other ways than those described herein, and similar improvements can be made by those skilled in the art without contradicting the intent of the disclosure. Therefore, the disclosure is not limited by specific embodiments disclosed below.

In the illustration of the embodiments of the disclosure, it may be understood that, the orientations or positional relationships indicated by the technical terms "center", "longitudinal", "transverse", "length", "width", "thickness", "upper", "lower", "front", "rear", "left", "right", "vertical", "horizontal", "top", "bottom", "inside", "outside", "clockwise", "counterclockwise", "axial", "radial", "circumferential", and the like are based on the orientations or positional relationships as illustrated in the accompanying drawings. These terms are merely for ease and brevity of illustration of the embodiments of the disclosure rather than indicating or implying that the means or components mentioned must have specific orientations or must be constructed or manipulated according to specific orientations, and therefore shall not be construed as any limitations on embodiments of the disclosure.

In addition, terms such as "first" and "second" are only used for descriptive purposes, and cannot be understood as indicating or implying relative importance or implicitly indicating the number of indicated technical features. Therefore, the features associated with "first" and "second" may explicitly or implicitly include at least one of the features. In the illustration of the embodiments of the disclosure, the term "multiple" means at least two, for example, two, three, and the like, unless otherwise specifically defined.

In the disclosure, unless otherwise specified and defined explicitly, the terms "mount", "connect", "join", and "fasten" may be understood in their general senses. For example, they may refer to a fixed connection, a detachable connection, or an integral connection, may refer to a mechanical connection or electrical connection, and may refer to a direct connection, an indirect connection via an intermediate medium, an internal communication between two elements, or an interaction between two elements, unless otherwise specifically defined. Persons of ordinary skill in the art may understand specific meanings of these terms in the disclosure as appropriate to specific situations.

In the disclosure, unless otherwise clearly specified and defined, a first feature being "on" or "under" a second feature may mean that the first and second features are in direct contact, or the first and second features are in indirect contact through an intermediary. Moreover, that the first feature is "above", "over", or "on" the second feature may mean that the first feature is directly above or obliquely above the second feature or simply mean that the first feature has a higher level than the second feature. That the first feature is "below", "beneath", and "under" the second feature may mean that the first feature is directly below or obliquely below the second feature or simply mean that the first feature has a lower level than the second feature.

It may be noted that, when an element is "fixed to" or "disposed on" another element, it may be directly on the other element or there may be a centered element. When an element is said to be "connected" to another element, it may be directly connected to the other element or there may be a centered element. As used herein, the terms "vertical", "horizontal", "top", "bottom", "left", "right", and similar expressions are used herein for illustrative purposes only and are not intended to be the only means of implementation.

In the related art, there is a blood pump capable of assisting the right ventricle in pumping blood. The delivery path for implanting such a blood pump generally starts from the inferior vena cava or superior vena cava, and sequentially passes through the right atrium, the tricuspid valve, the right ventricle, and the pulmonary valve to reach the pulmonary artery. This right ventricular delivery path is characterized by its short path, numerous bends, and complex internal tissue structures. Consequently, during implantation, the blood pump often needs to make multiple turns to navigate these bends of the delivery path.

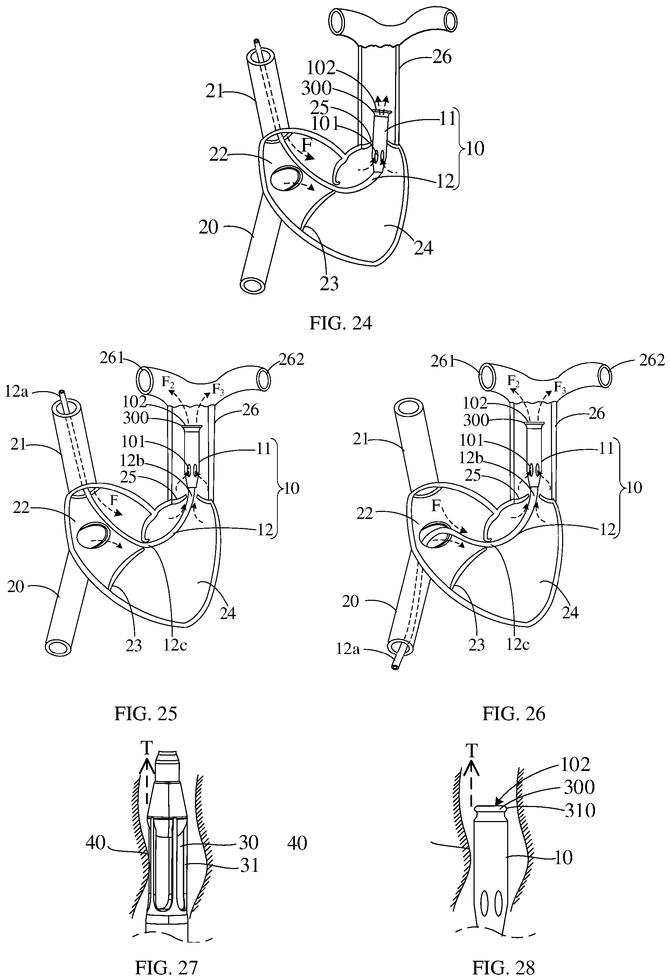

Referring to FIG. 27, a conventional blood pump is typically provided with multiple side openings 30 on the distal side wall thereof, and these side openings 30 serve as liquid outlets. The multiple side openings 30 are spaced apart from each other in a circumferential direction of the blood pump, with a connecting column 31 formed between two adjacent side openings 30. These connecting columns 31 are elongated and are made of metal, rendering a peripheral edge of the side openings 30 relatively sharp. Thus, due to the short path and numerous bends of the right ventricular delivery path, when the blood pump is being implanted, a distal end of the blood pump often needs to undergo a significant change in direction as the blood pump turns through a narrow bend. Consequently, the distal side surface of the blood pump is highly likely to abut against the inner tissue wall at the bend. The inner tissue wall may invaginate into the side openings 30 of the blood pump. When the blood pump is subsequently pushed further in the direction indicated by *T* as illustrated in FIG. 27, the connecting columns 31 on both sides of the side openings 30 scrape against the portion of the inner tissue wall 40 invaginated into the side openings 30, causing damage to a patient's internal tissue.

Referring to FIG. 28, to solve the above problem, the disclosure provides a blood pump 10. The blood pump 10 includes a pump body 11 and a catheter 12. The blood pump 10 can reduce collision or friction between the pump body 11 and the inner tissue wall 40 during implantation, so as to avoid damage to a patient's internal tissue. The blood pump 10 mainly serves as a right ventricular assist pump. In other embodiments, the blood pump 10 may serve as a left ventricular assist pump. It may be noted that, in the field of medical equipment technology, the end of the medical equipment close to the physician or operator is generally referred to as the proximal end, and the end away from the physician or operator is referred to as the distal end.

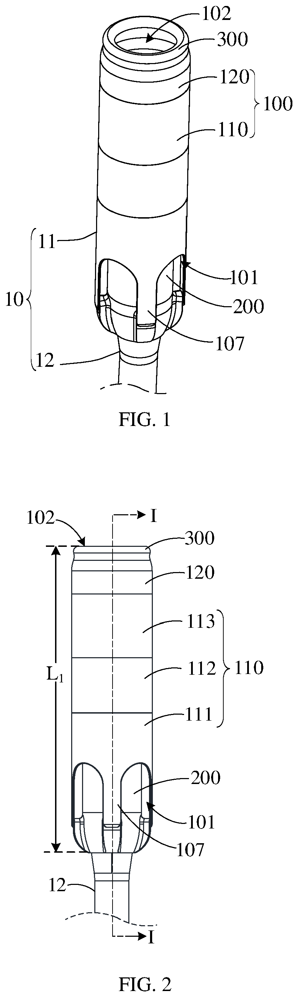

Referring to FIGS. 1 - 3, in an embodiment of the blood pump 10 of the disclosure, the blood pump 10 includes a pump body 11, and the pump body 11 includes a pump casing 100 and a driving unit 200. A proximal end of the pump casing 100 is provided with a liquid inlet 101, a distal end of the pump casing 100 is open to define the distal end of the pump casing 100 as a liquid outlet 102, and a central axis *M*1*M*2 of the pump casing 100 passes through the liquid outlet 102. The driving unit 200 is fixedly connected to the proximal end of the pump casing 100, and the driving unit 200 is configured to drive blood to flow from the liquid inlet 101 to the liquid outlet 102.

Configuring the open distal end of the pump casing 100 as the liquid outlet 102 may ensure that no opening is formed on the distal side wall of the pump casing 100. Thus, during implantation of the blood pump 10, when the pump body 11 of the blood pump 10 turns through a narrow bend of the delivery path, even if the distal side wall of the pump body 11 abuts against the inner tissue wall at the bend, the inner tissue wall may not radially invaginate into the pump body 11. Compared with the side wall of the pump casing 100, the open distal end of the pump casing 100 is less likely to directly contact the inner tissue wall. Therefore, configuring the open distal end of the pump casing 100 as the liquid outlet 102 can reduce the likelihood that the inner tissue wall invaginates into the liquid outlet 102. In addition, the flow area of the liquid outlet 102 is relatively large, which can increase the blood pumping volume.

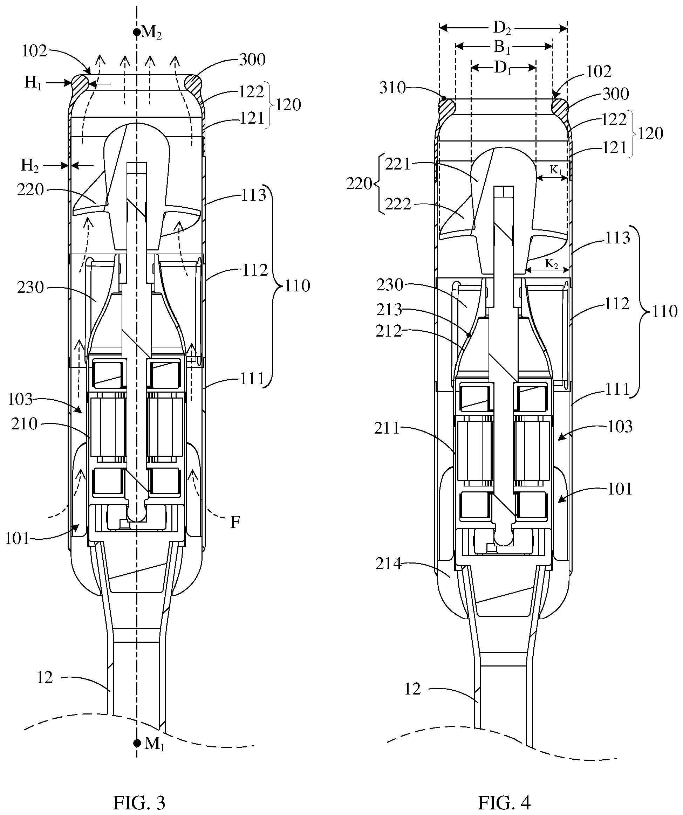

Furthermore, the disclosure takes into consideration that the wall thickness of the pump casing 100 is generally relatively small, so that a peripheral edge of the liquid outlet 102 of the pump casing 100 may be relatively sharp. As illustrated in FIG. 28, during implantation of the blood pump 10, when the blood pump 10 is pushed in the direction indicated by *T* as illustrated in FIG. 28, the peripheral edge of the liquid outlet 102 of the pump casing 100 may come into contact with the inner tissue wall. Therefore, to prevent the periphery of the liquid outlet 102 of the pump casing 100 from scratching the inner tissue wall, in the disclosure, the pump body 11 is further provided with an atraumatic ring 300. The atraumatic ring 300 is disposed around the peripheral edge of the liquid outlet 102. As illustrated in FIG. 3 and FIG. 4, a radial thickness *H*1 of the atraumatic ring 300 is greater than a wall thickness *H*2 of the pump casing 100, i.e., *H*1 > *H*2. The atraumatic ring 300 is provided with an annular wall 310 extending in a circumferential direction of the atraumatic ring 300, and a center of a generating circle of a torus in which the annular wall 310 is located is positioned inside the atraumatic ring 300.

Specifically, setting the radial thickness *H*1 of the atraumatic ring 300 to be greater than the wall thickness *H*2 of the pump casing 100 can increase the radial thickness of the atraumatic ring 300, thereby enhancing the atraumatic property of the atraumatic ring 300 and making the atraumatic ring 300 less sharp. Moreover, the annular wall 310 extending in the circumferential direction of the atraumatic ring 300 is provided on the surface of the atraumatic ring 300, which can make the surface of the atraumatic ring 300 more rounded and smooth, thereby reducing the likelihood of scratching the inner tissue wall. The annular wall 310 is located on a torus. The torus refers to a ring-shaped structure formed by rotating a circle around an axis that does not intersect the circle. The circle is generally referred to as the generating circle of the torus. For example, structures such as swimming rings, doughnuts, and hula hoops all have toroidal shapes. The atraumatic ring 300 may be entirely configured as a torus, so that the entire surface of the atraumatic ring 300 forms the annular wall 310. Alternatively, the atraumatic ring 300 may not be a torus, but only a portion of the surface of the atraumatic ring 300 is configured as the annular wall 310, and the torus in which the annular wall 310 is located is a virtual torus used to design the shape of the annular wall 310.

Regardless of whether the torus in which the annular wall 310 is located is the atraumatic ring 300 or a virtual torus, the center of the generating circle of the torus is positioned inside the atraumatic ring 300, so that the annular wall 310 protrudes outwardly relative to the interior of the atraumatic ring 300, thereby making the periphery of the atraumatic ring 300 relatively rounded and smooth. When the blood pump 10 is implanted into a patient's body, the distal end of the pump body 11 comes into contact with the inner tissue wall via the atraumatic ring 300. Since the annular wall 310 of the atraumatic ring 300 is relatively rounded and smooth, the inner tissue wall is less likely to be scratched, thereby avoiding damage to the patient's internal tissue.

In the technical solution of the disclosure, configuring the open distal end of the pump casing 100 as the liquid outlet 102 may ensure that no opening is formed on the distal side wall of the pump casing 100. Thus, during implantation of the blood pump 10, when the pump body 11 of the blood pump 10 turns through a narrow bend of the delivery path, even if the distal side wall of the pump body 11 abuts against the inner tissue wall at the bend, the inner tissue wall may not radially invaginate into the pump body 11. Furthermore, in the disclosure, the atraumatic ring 300 is disposed around the peripheral edge of the liquid outlet 102 of the pump casing 100. The radial thickness *H*1 of the atraumatic ring 300 is greater than the wall thickness *H*2 of the pump casing 100. The atraumatic ring 300 is provided with the annular wall 310 extending in the circumferential direction of the atraumatic ring 300, and the center of the generating circle of the torus in which the annular wall 310 is located is positioned inside the atraumatic ring 300, so that the annular wall 310 protrudes outwardly relative to the interior of the atraumatic ring 300. In this way, the periphery of the atraumatic ring 300 is relatively rounded and smooth, preventing the peripheral edge of the liquid outlet 102 from being excessively sharp, so that the liquid outlet 102 is less likely to scratch the inner tissue wall, thereby preventing the distal end of the pump body 11 from scratching a patient's internal tissue.

Referring to FIG. 2 and FIG. 3, when the blood pump 10 is operating, the driving unit 200 drives blood to enter the pump casing 100 through the liquid inlet 101. The blood continues to be driven by the driving unit 200 in the pump casing 100 and flows toward the liquid outlet 102 in an axial direction of the driving unit 200 (i.e., the direction from *M*1 to *M*2). When the blood flows to the distal end of the pump casing 100, the blood can be directly discharged through the liquid outlet 102 in the axial direction of the driving unit 200. In this way, the blood does not undergo a significant radial turn when being discharged through the liquid outlet 102, thereby reducing kinetic energy loss during blood discharge.

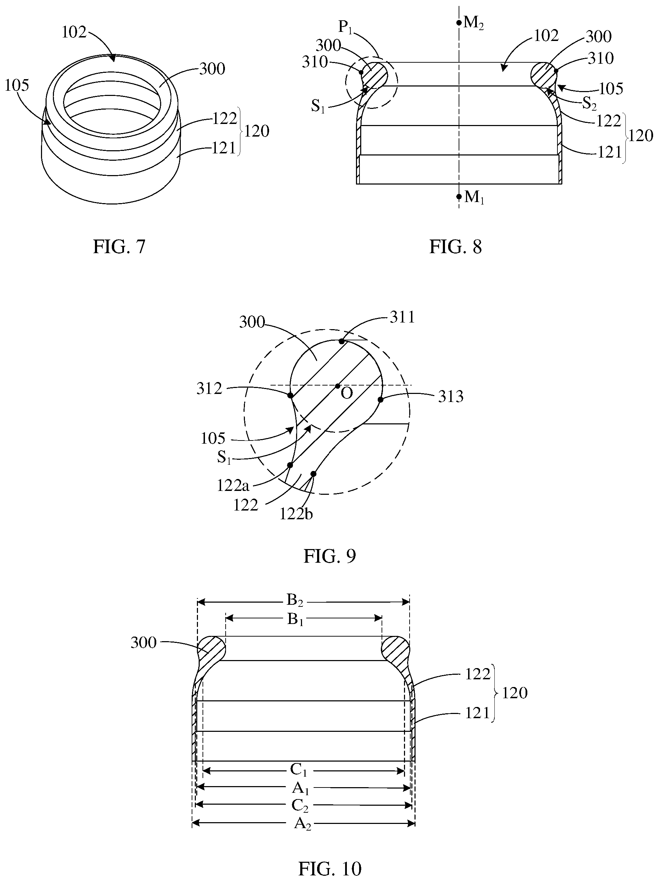

Referring to FIG. 3 and FIGS. 7 - 9, a distal end of the atraumatic ring 300 is more likely to come into frictional contact with the inner tissue wall. Therefore, in this embodiment, the annular wall 310 of the atraumatic ring 300 at least includes a distal side wall 311 located at the most distal end of the atraumatic ring 300. A cross-section of the distal side wall 311 taken along an axial plane is semicircular, and the axial plane is a plane passing through the central axis. In FIG. 9, point *O* represents the center of the circle where the cross-section of the distal side wall 311 is located. That is, point *O* represents the center of the generating circle of the torus in which the distal side wall 311 is located.

Since the atraumatic ring 300 has a radial thickness *H*1, the generating circle of the torus in which the distal side wall 311 is located may be a circle having a diameter equal to the radial thickness *H*1 of the atraumatic ring 300. Thus, the cross-section of the distal side wall 311 taken along the axial plane is semicircular. With this configuration, the distal side wall 311 forms the distal end surface of the atraumatic ring 300, and opposite sides of the distal side wall 311 smoothly transition to the inner peripheral surface of the pump casing 100 and the outer peripheral surface of the pump casing 100, respectively. Thus, when the distal end of the atraumatic ring 300 contacts the inner tissue wall, the atraumatic ring 300 is less likely to scratch the inner tissue wall, thereby reducing the risk of implanting the blood pump 10.

Referring to FIG. 3, FIG. 8, and FIG. 9, optionally, the annular wall 310 further includes an outer side wall 312 located on an outer side of the atraumatic ring 300. A cross-section of the outer side wall 312 taken along the axial plane is arc-shaped. The outer side wall 312 smoothly connects the distal side wall 311 and an outer peripheral surface of the pump casing 100. The outer side wall 312 forms a part of the outer side surface of the atraumatic ring 300, so that outer side surface of the atraumatic ring 300 is relatively smooth. Thus, when the outer side surface of the atraumatic ring 300 contacts the inner tissue wall, the outer side surface of the atraumatic ring 300 is less likely to scratch the inner tissue wall, thereby reducing the risk of implanting the blood pump 10.

Furthermore, the annular wall 310 further includes an inner side wall 313 located on an inner side of the atraumatic ring 300. A cross-section of the inner side wall 313 taken along the axial plane is arc-shaped. The inner side wall 313 smoothly connects the distal side wall 311 and an inner peripheral surface of the pump casing 100. The inner side wall 313 forms a part of the inner side surface of the atraumatic ring 300, leading to a smoother inner side surface of the atraumatic ring 300. When blood is discharged through the liquid outlet 102, a portion of the blood contacts the inner side wall 313. The inner side wall 313 is less likely to damage blood cells, thereby improving the operational safety of the blood pump 10.

Referring to FIGS. 7 - 9, in an embodiment, the atraumatic ring 300 is configured as a torus, and the atraumatic ring 300 serves as the torus in which the annular wall 310 is located. Furthermore, two cross-sections *S*1 and *S*2 of the atraumatic ring 300 taken along any axial plane are symmetrical with respect to the central axis *M*1*M*2. That is, either of the cross-sections *S*1 and *S*2 of the atraumatic ring 300 may serve as the generating circle of the torus, and the center of the generating circle is point *O*. Thus, two cross-sections of the atraumatic ring 300 taken along any axial plane are symmetrical with respect to the central axis *M*1*M*2. In this way, the entire atraumatic ring 300 is relatively smooth in the circumferential direction of the atraumatic ring 300, without forming sharp edges, thereby greatly reducing the risk of the pump casing 100 damaging the inner tissue wall.

Referring to FIGS. 1 - 3, the atraumatic ring 300 and the pump casing 100 may be integrally formed, or the atraumatic ring 300 may be separated from the pump casing 100. Specifically, the atraumatic ring 300 may be correspondingly designed according to the specific structure of the pump casing 100. Optionally, in an embodiment, the pump casing 100 includes a casing tube 110 fixedly connected to the driving unit 200 and includes an extension tube 120 fixedly connected to the casing tube 110. The casing tube 110 is provided with the liquid inlet 101. A proximal end of the extension tube 120 is fixedly connected to the casing tube 110, and a distal end of the extension tube 120 is provided with a distal open end that defines the liquid outlet 102. The atraumatic ring 300 is connected to a peripheral edge of the distal open end, and the atraumatic ring 300 constitutes the most distal end of the blood pump 10.

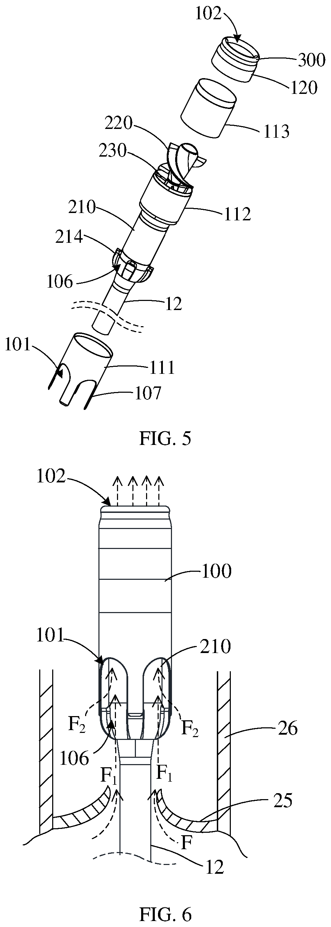

Referring to FIG. 3 and FIG. 5, the casing tube 110 and the extension tube 120 are two separate components. The atraumatic ring 300 may be integrally formed with the extension tube 120 to reduce the number of components and reduce processing and assembly procedures. However, in other embodiments, the atraumatic ring 300 may be manufactured separately and then fixedly connected to the extension tube 120. Specifically, in this embodiment, the atraumatic ring 300 is integrally formed with the extension tube 120.

In an embodiment, at least one of the extension tube 120 and the atraumatic ring 300 is of an elastic structure. For example, the atraumatic ring 300 is of an elastic structure, so that the atraumatic ring 300 is elastic. In this way, during implantation of the blood pump 10, the atraumatic ring 300 can elastically deform when the atraumatic ring 300 contacts the inner tissue wall, thereby buffering the force exerted by the atraumatic ring 300 on the inner tissue wall.

Similarly, the extension tube 120 may be of an elastic structure. During implantation of the blood pump 10, when the atraumatic ring 300 contacts the inner tissue wall, the force between the atraumatic ring 300 and the inner tissue wall is transmitted to the extension tube 120, and the elastic deformation of the extension tube 120 can buffer the force exerted by the atraumatic ring 300 on the inner tissue wall. Since the atraumatic ring 300 and the extension tube 120 are integrally formed, in this embodiment, both the atraumatic ring 300 and the extension tube 120 are of an elastic structure. The elastic structure refers to a structure supported by an elastic material, and the elastic material may be a thermoplastic polyurethane (TPU) material. However, in other embodiments, the atraumatic ring 300 and the extension tube 120 may be made of metal material, so that the atraumatic ring 300 and the extension tube 120 have rigidity.

Referring to FIG. 3 and FIG. 8, in an embodiment, since the radial thickness *H*1 of the atraumatic ring 300 is greater than the wall thickness *H*2 of the pump casing 100, in order to avoid forming a relatively sharp transition step at the junction between the pump casing 100 and the atraumatic ring 300, optionally, the extension tube 120 includes a connecting section 121 fixedly connected to the casing tube 110 and includes a transition section 122 connecting the connecting section 121 and the atraumatic ring 300. The inner peripheral surface of the connecting tube 113 is axially aligned and flush with the inner peripheral surface of the casing tube 110. The inner and outer peripheral surfaces of the connecting tube 113 are axially aligned and flush with the outer peripheral surface of the casing tube 110. The pump casing 100 gradually transitions to the atraumatic ring 300 via the transition section 122, so that the junction between the pump casing 100 and the atraumatic ring 300 is relatively smooth and is less likely to form a sharp transition step.

Referring to FIG. 3 and FIG. 10, further, the connecting section 121 has a first inner diameter *A*1, and the atraumatic ring 300 has a second inner diameter *B*1. The second inner diameter *B*1 is the minimum inner diameter of the atraumatic ring 300, and the second inner diameter *B*1 is smaller than the first inner diameter *A*1, i.e., *B*1 < *A*1. In this way, the liquid outlet 102 forms a contracted opening relative to the inner cavity of the connecting section 121. When blood is discharged through the liquid outlet 102, the blood is compressed so that the blood pressure increases, thereby enabling the blood to obtain greater potential energy after the blood is discharged through the liquid outlet 102 to a pulmonary artery 26, and thus increasing the flow velocity of the blood in the pulmonary artery 26.

Still referring to FIG. 3 and FIG. 10, optionally, the connecting section 121 has a first outer diameter *A*2, and the atraumatic ring 300 has a second outer diameter *B*2. The second outer diameter *B*2 is the maximum outer diameter of the atraumatic ring 300, and the second outer diameter *B*2 is smaller than the first outer diameter *A*2, i.e., *B*2 < *A*2. Since the atraumatic ring 300 forms the most distal end of the pump body 11, setting the second outer diameter *B*2 to be smaller than the first outer diameter *A*2 is equivalent to reducing the diameter of the most distal end of the pump body 11. This can reduce the resistance encountered when the most distal end of the pump body 11 passes through various bends along the delivery path, thereby allowing the pump body 11 to be implanted into a patient's body more smoothly.

Referring to FIGS. 8 - 10, in an embodiment, the transition section 122 has an inner transition surface 122b and an outer transition surface 122a. The outer transition surface 122a connects the outer peripheral surface of the connecting section 121 and the outer side wall 312 of the atraumatic ring 300. The inner transition surface 122b connects the inner peripheral surface of the connecting section 121 and the inner side wall 313 of the atraumatic ring 300. The transition section 122 has a third inner diameter *C*1, which is the diameter of the inner transition surface 122b. The third inner diameter *C*1 gradually decreases in a direction from the connecting section 121 to the atraumatic ring 300. In this way, the inner transition surface 122b may smoothly connect the inner peripheral surface of the connecting section 121 and the inner side wall 313 of the atraumatic ring 300, leading to a smoother inner peripheral surface of the pump casing 100, thereby reducing blood flow resistance and minimizing damage to blood cells.

Still referring to FIGS. 8 - 10, further, the transition section 122 has a third outer diameter *C*2, which is the diameter of the outer transition surface 122a. The third outer diameter *C*2 first gradually decreases and then gradually increases in the direction from the connecting section 121 to the atraumatic ring 300. In this way, the outer transition surface 122a may smoothly connect the outer peripheral surface of the connecting section 121 and the outer side wall 312 of the atraumatic ring 300, leading to a smoother outer peripheral surface of the pump casing 100, thereby avoiding the formation of sharp transition steps on the outer peripheral surface of the pump casing 100, and thus reducing collision damage with the inner tissue wall.

Since the third outer diameter *C*2 first gradually decreases and then gradually increases in the direction from the connecting section 121 to the atraumatic ring 300, an annular recess 105 is formed on the outer side of the transition section 122. In the case where the atraumatic ring 300 is of an elastic structure, the presence of the annular recess 105 can enhance the amplitude of elastic deformation of the atraumatic ring 300 in the axial direction, further buffering the reaction force exerted by the atraumatic ring 300 on the inner tissue wall.

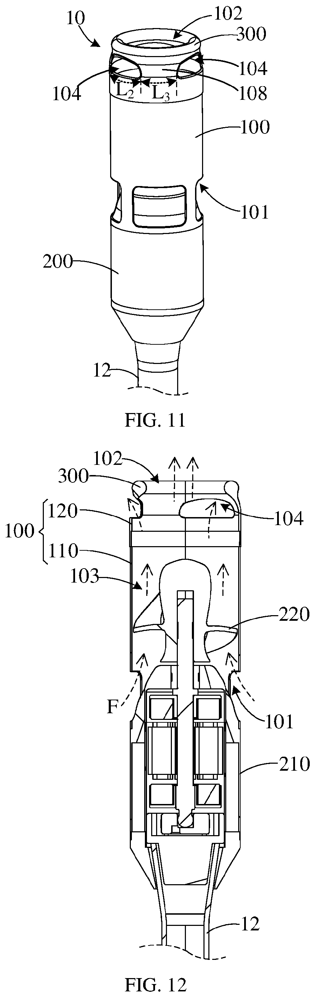

In the embodiment of the blood pump 10 illustrated in FIG. 11 and FIG. 12, since the transition section 122 is the transition portion of the extension tube 120, the transition section 122 is recessed toward the central axis *M*1*M*2 of the pump casing 100 relative to the connecting section 121 and the atraumatic ring 300, so that the transition section 122 is less likely to come into surface contact with the inner tissue wall. Therefore, optionally, the transition section 122 is provided with multiple through holes 104 penetrating through the side wall of the transition section 122, and the multiple through holes 104 are spaced apart in the circumferential direction of the transition section 122. A portion of the blood in the pump casing 100 can be directly discharged axially through the liquid outlet 102, while a small portion of the blood can be discharged radially through the through holes 104, thereby increasing the amount of blood pumped by the blood pump 10.

Since the multiple through holes 104 are arranged at intervals, a partition wall 108 is formed between each two adjacent through holes 104. To prevent the partition wall 108 from forming a prismatic shape, optionally, each of the multiple through holes 104 has a second length *L*2 extending in the circumferential direction of the extension tube 120, and the partition wall 108 has a third length *L*3 extending in the circumferential direction of the extension tube 120. The third length *L*3 is greater than or equal to the second length *L*2, i.e., *L*3 ≥ *L*2. With this configuration, the partition wall extends a longer distance in the circumferential direction of the extension tube 120, thereby providing a relatively large surface area and preventing the partition wall from forming a prismatic shape. As a result, even if the inner tissue wall comes into contact with the through hole 104 or the partition wall 108, the through hole 104 or the partition wall 108 is less likely to damage the inner tissue wall.

Regarding the driving unit 200 of the blood pump 10, the driving unit 200 includes a motor 210 and an impeller 220 connected to the motor 210. The motor 210 is configured to drive the impeller 220 to rotate, so that the impeller 220 drives blood to flow into the pump casing 100 through the liquid inlet 101 and be discharged through the liquid outlet 102 of the pump casing 100. In the disclosure, there are two ways to assemble the driving unit 200 and the pump casing 100. For example, referring to FIG. 11 and FIG. 12, in an embodiment, only the impeller 220 of the driving unit 200 is disposed inside the pump casing 100, a distal end of the motor 210 is fixedly connected to the proximal end of the pump casing 100, and a shaft of the motor 210 extends into the pump casing 100 to be fixedly connected to the impeller 220. In this way, a first length of the pump body 11 is approximately equal to the sum of the length of the motor 210 and the length of the pump casing 100.

In the embodiment illustrated in FIGS. 1 - 6, both the motor 210 and the impeller 220 of the driving unit 200 are disposed inside the pump casing 100. The motor 210 is spaced apart from the inner wall surface of the pump casing 100 to define a blood flow passage 103, and the blood flow passage 103 communicates the liquid outlet 102 with the liquid inlet 101. A proximal end of the motor 210 is fixedly connected to the proximal end of the pump casing 100. Specifically, the proximal end of the pump casing 100 is fixedly connected to the proximal end of the motor 210, and the inner wall surface of the pump casing 100 is spaced apart from the outer peripheral surface of the motor 210 to form a part of the blood flow passage 103. In this way, the first length *L*1 of the pump body 11 is approximately equal to the length of the pump casing 100, which may ensure that the first length *L*1 of the pump body 11 is relatively small, ensuring that the first length *L*1 of the pump body 11 is suitable for both the delivery path to a right ventricle 24 and the anatomical structure of the pulmonary artery 26, thereby facilitating implantation into the pulmonary artery 26.

Referring to FIG. 3, FIG. 6, and FIG. 7, optionally, the proximal end of the pump casing 100 is provided with multiple connecting arms 107, and each two adjacent connecting arms 107 are spaced apart from each other to define the liquid inlet 101. Multiple fixing portions 214 protrude from the proximal end of the outer peripheral surface of the motor 210, and every two adjacent fixing portions 214 are spaced apart to define an inlet groove 106. The inlet groove 106 faces the proximal end of the catheter 12. The multiple fixing portions 214 are connected to the multiple connecting arms 107 in a one-to-one correspondence, so that the inlet groove 106 is connected to and communicates with the proximal end of the liquid inlet 101. When the pump body 11 is disposed in the pulmonary artery 26, the liquid inlet 101 of the pump body 11 is adjacent to a pulmonary valve 25, the inlet groove 106 faces the central region of the pulmonary valve 25, and the catheter 12 passes through the central region of the pulmonary valve 25. During operation of the blood pump 10, after blood from the right ventricle 24 passes through the pulmonary valve 25, a portion of the blood *F*2 is drawn radially from the periphery of the pump body 11 into the liquid inlet 101, and then driven by the driving unit 200 to switch to axial flow into the blood flow passage 103. Another portion of the blood *F*1 can be guided by a distal end of the catheter 12 to flow axially into the inlet groove 106, and then be axially guided through the inlet groove 106 into the inner side of the liquid inlet 101, thereby pushing the portion of the blood *F*2 entering through the liquid inlet 101 to flow axially. In this way, the blood *F*2 is promoted to accelerate into the blood flow passage 103, thereby effectively increasing the blood flow rate entering through the liquid inlet 101 and improving the pumping efficiency of the blood pump 10.

Referring to FIG. 3 and FIG. 4, optionally, the pump casing 100 includes the casing tube 110, and both the motor 210 and the impeller 220 are located inside the casing tube 110. The casing tube 110 includes an inlet tube 111, a fixing tube 112, and a connecting tube 113. The inlet tube 111 and the connecting tube 113 are respectively connected to two ends of the fixing tube 112. The inlet tube 111 is provided with the liquid inlet 101, and an end of the inlet tube 111 away from the fixing tube 112 is fixedly connected to the proximal end of the motor 210. An inner wall surface of the fixing tube 112 is fixedly connected to the outer peripheral surface of the motor 210 via a support member.

Specifically, the fixing tube 112 is sleeved around the outer periphery of the motor 210, and the inner wall surface of the fixing tube 112 is spaced apart from the outer peripheral surface of the motor 210. The inner wall surface of the fixing tube 112 is provided with multiple support members, and the multiple support members are fixedly connected to the outer peripheral surface of the motor 210 to support and secure the fixing tube 112. The fixing tube 112 forms the side wall of the middle portion of the casing tube 110. Therefore, the support members stably support and secure the fixing tube 112, so that the fixing tube 112 serves a central supporting role in the casing tube 110, ensuring that both the inlet tube 111 and the connecting tube 113 maintain stable relative positions with respect to the outer surface of the motor 210, thereby improving the structural stability of the blood flow passage 103 formed between the casing tube 110 and the driving unit 200.

It may be noted that, the shape and structure of the above-mentioned support member are not specifically limited. The support member may be a support rib extending in the radial direction of the motor 210, or a flow-guiding plate extending in the axial direction of the motor 210. During assembly, the inlet tube 111 of the casing tube 110 may be sleeved from the proximal end of the motor 210 onto the outer periphery of the proximal end of the motor 210 and fixedly connected to the proximal end of the fixing tube 112, and the connecting tube 113 of the casing tube 110 may be sleeved from the distal end of the motor 210 onto the outer periphery of the distal end of the motor 210 and fixedly connected to the distal end of the fixing tube 112. In this way, the casing tube 110 is connected and fixed to the motor 210, thereby improving the convenience of assembly.

Referring to FIG. 3 and FIG. 4, optionally, the motor 210 includes a motor body 211 and a shaft support base 212 connected to a distal end of the motor body 211. The outer diameter of the shaft support base 212 gradually decreases in a direction from the motor 210 to the impeller 220, such that the outer peripheral surface of the shaft support base 212 forms a conical flow-guiding surface 213. The conical flow-guiding surface 213 can guide the blood passing through the outer periphery of the motor 210 to gradually deflect and flow toward the central axis of the impeller 220, and then flow toward the central region of the impeller 220. That is, the shaft support base 212 can guide the blood gradually toward the central region of the impeller 220 to accommodate the radial size difference between the motor 210 and a hub 221 of the impeller 220.

Further, multiple rectifying plates 230 are disposed on the conical flow-guiding surface 213. The multiple rectifying plates 230 are arranged at intervals in a circumferential direction of the conical flow-guiding surface 213, and the multiple rectifying plates 230 all extend in an axial direction of the motor 210. The rectifying plates 230 and the shaft support base 212 may be integrally formed. Specifically, the multiple rectifying plates 230 are fixed to the conical flow-guiding surface 213 at intervals in the circumferential direction of the shaft support base 212, so as to form a linearly extending rectifying channel between two adjacent rectifying plates 230. That is, the rectifying channels extend linearly in the axial direction of the motor 210, so as to guide the blood in the blood flow passage 103 to flow axially into the impeller 220.

Thus, every two of the multiple rectifying plates 230 define a rectifying channel, so that a rectifying device 400 has multiple linearly extending rectifying channels, and the multiple rectifying channels are spaced apart in the circumferential direction of the shaft support base 212. When blood flows to the outer periphery of the shaft support base 212, the blood is divided by the multiple rectifying plates 230 into multiple strands of ordered blood flow stably flowing in the axial direction, and these ordered blood flows flow axially into the impeller 220.

In addition, a side edge of the rectifying plate 230 away from the conical flow-guiding surface 213 may be fixedly connected to the inner wall surface of the fixing tube 112, so that the rectifying plate 230 serves as the support member to support the fixing tube 112.

Referring to FIG. 3 and FIG. 4, based on any of the above embodiments, the impeller 220 of the driving unit 200 includes the hub 221 and a blade 222 disposed on the hub 221. The diameter of the hub 221 gradually increases in a direction from the motor 210 to the impeller 220. In this way, the distance between the outer peripheral surface of the hub 221 and the inner peripheral surface of the pump casing 100 gradually decreases in a diameter-expanding direction of the hub 221. The diameter-expanding direction is a direction from the motor 210 to the impeller 220. With such an arrangement, a distal end of the hub 221 has a larger diameter, so that a distance *K*2 between the outer peripheral surface of the distal end of the hub 221 and the inner peripheral surface of the pump casing 100 is smaller than a distance *K*1 between the outer peripheral surface of a proximal end of the hub 221 and the inner peripheral surface of the pump casing 100, i.e., *K*2 < *K*1.

Blood enters the impeller 220 at the location of distance *K*1 and is driven by the impeller 220 into a spiral rotation, thereby being gradually pushed by the impeller 220 toward the location of distance *K*2. As the path from the position of distance *K*1 to the position of distance *K*2 gradually narrows, the blood is gradually compressed spirally during this process. As a result, due to spiral driving force exerted by the impeller 220, a portion of the radial potential energy gained by the blood is converted into axial potential energy, increasing the axial potential energy of the blood, which in turn increases the axial velocity component of the blood, allowing the blood to be quickly discharged axially from the liquid outlet 102.

The diameter of the hub 221 gradually increases in the direction from the motor 210 to the impeller 220, such that the distal end of the hub 221 has a fourth outer diameter *D*1, and the fourth outer diameter *D*1 is the maximum diameter of the hub 221. The impeller 220 has a fifth outer diameter *D*2, and the fifth outer diameter *D*2 is the maximum diameter of the impeller 220 during rotation of the impeller 220 as a whole. The atraumatic ring 300 has a second inner diameter *B*1, and the second inner diameter *B*1 is the minimum inner diameter of the atraumatic ring 300. Optionally, the second inner diameter *B*1 is greater than the fourth outer diameter *D*1 and smaller than the fifth outer diameter *D*2, i.e., *D*1 < *B*1 < *D*2.

Specifically, the second inner diameter *B*1 corresponds to the diameter of the liquid outlet 102. The second inner diameter *B*1 being smaller than the fifth outer diameter *D*2 means that the diameter of the liquid outlet 102 is smaller than the maximum diameter of the impeller 220, so that the liquid outlet 102 forms a contracted opening relative to the blood flow passage 103. When blood is discharged through the liquid outlet 102, the blood is compressed, causing the blood pressure to rise, so that the blood gains greater potential energy after being discharged into the pulmonary artery 26, thereby increasing the blood flow velocity in the pulmonary artery 26. The second inner diameter *B*1 being greater than the fourth outer diameter *D*1 means that the diameter of the liquid outlet 102 is greater than the maximum diameter of the distal end of the hub 221. Consequently, when the blood guided by the distal end of the hub 221 is discharged axially, the blood does not directly impinge on the peripheral edge of the liquid outlet 102, thereby reducing the kinetic energy loss of the blood, increasing the discharge velocity of the blood, and improving pumping efficiency.

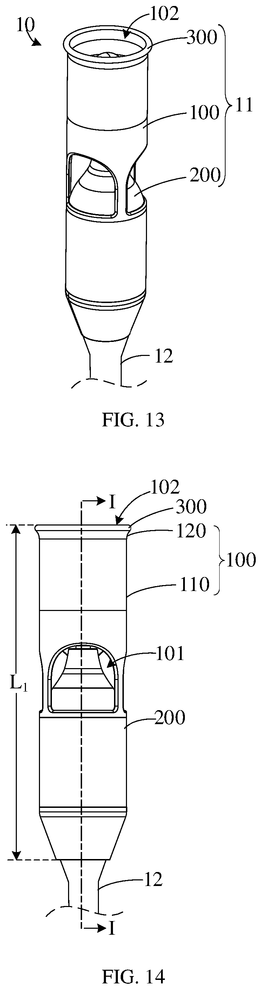

FIGS. 13 - 23 show another embodiment of the blood pump 10 provided in the disclosure. Referring to FIGS. 13 - 15, in this embodiment, the blood pump 10 includes a pump body 11 and a catheter 12, and the catheter 12 is connected to the proximal end of the pump body 11. The pump body 11 includes a pump casing 100 and a driving unit 200. The pump casing 100 is provided with a liquid inlet 101. The distal end of the pump casing 100 is open to define the distal end of the pump casing 100 as a liquid outlet 102. The driving unit 200 includes an impeller 220 disposed inside the pump casing 100, and the impeller 220 faces the liquid outlet 102.

Referring to FIG. 24, when implanting the blood pump 10 into a patient's body, the pump body 11 of the blood pump 10 may be advanced from an inferior vena cava 20 or a superior vena cava 21, sequentially through a right atrium 22, a tricuspid valve 23, a right ventricle 24, and a pulmonary valve 25 to reach a pulmonary artery 26, so that at least the liquid outlet 102 of the pump body 11 extends into the pulmonary artery 26. During operation of the blood pump 10, the driving unit 200 drives the impeller 220 to rotate, causing blood to enter the pump casing 100 through the liquid inlet 101. The blood is then accelerated by the impeller 220 in the pump casing 100 and finally discharged outward through the liquid outlet 102 into the pulmonary artery 26, thereby assisting the right ventricle 24 in pumping blood to the pulmonary artery 26. Obviously, the blood in the pump casing 100 flows in the axial direction of the impeller 220.

In the disclosure, the impeller 220 faces the liquid outlet 102, i.e., the liquid outlet 102 is axially opposite to the impeller 220. Consequently, when blood flows axially along the impeller 220 to the liquid outlet 102, the blood continues to be discharged axially along the impeller 220 from the liquid outlet 102. Thus, the blood is substantially not obstructed by other components, and the flow direction of most of the blood does not undergo a significant change. As a result, the kinetic energy loss of the discharged blood is relatively low, allowing the blood to be discharged through the liquid outlet 102 more quickly, which facilitates an increase in the volume of blood discharged through the liquid outlet 102. Furthermore, since the liquid outlet 102 is opposite to the impeller 220 and is not located on the side wall of the pump casing 100, when the side wall of the pump body 11 accidentally abuts against the inner tissue wall, the liquid outlet 102 is less likely to be blocked by the inner tissue wall and can normally discharge blood.

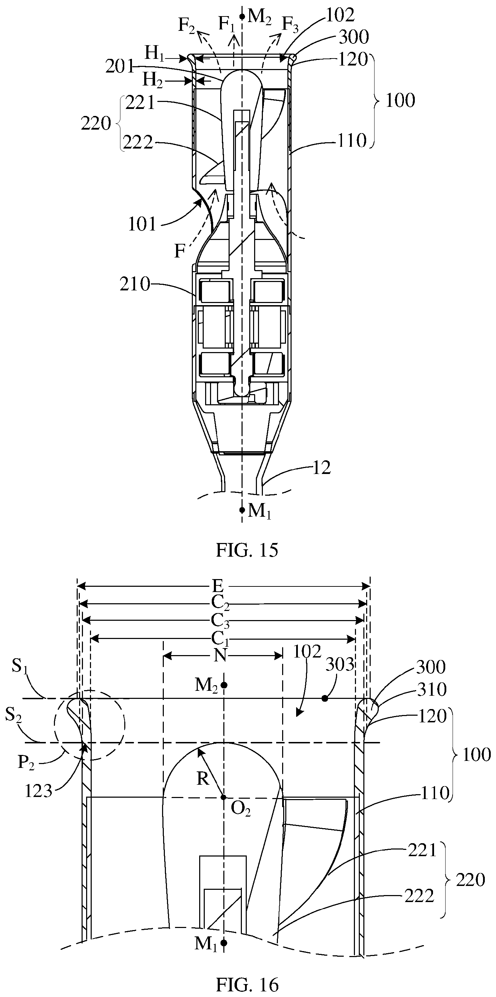

Referring to FIG. 15 and FIG. 16, the disclosure takes into consideration that the wall thickness *H*2 of the pump casing 100 is generally relatively small, and the width of the distal end surface of the pump casing 100 (i.e., the peripheral edge of the liquid outlet 102) extending in the radial direction of the pump casing 100 is substantially equal to *H*2, so that the peripheral edge of the liquid outlet 102 is relatively sharp. During implantation of the blood pump 10 into a patient's body, the blood pump 10 is pushed into the patient's body in the direction from the catheter 12 to the pump body 11, and the peripheral edge of the liquid outlet 102 of the pump body 11 may come into contact with and scratch the inner tissue wall. In view of this, to reduce the possibility that the peripheral edge of the liquid outlet 102 scratches the inner tissue wall, in the disclosure, the pump body 11 is further provided with an atraumatic ring 300. The atraumatic ring 300 is disposed around the peripheral edge of the distal end of the pump casing 100. At least a distal end of the atraumatic ring 300 is provided with an atraumatic wall 310. The atraumatic wall 310 extends in a circumferential direction of the atraumatic ring 300, and the atraumatic wall 310 has a radial width *H*1 in a radial direction of the pump casing 100. The radial width *H*1 is greater than a wall thickness *H*2 of the pump casing 100, i.e., *H*1 > *H*2.

Specifically, the atraumatic wall 310 extends in the circumferential direction of the atraumatic ring 300. The circumferential direction of the atraumatic ring 300 refers to the direction surrounding the central axis *M*1*M*2 of the impeller 220. By setting the radial width *H*1 of the atraumatic wall 310 to be greater than the wall thickness *H*2 of the pump casing 100, the radial width *H*1 of the atraumatic wall 310 is ensured to be relatively large, so that the surface area of the atraumatic wall 310 is increased and the atraumatic property of the atraumatic ring 300 is enhanced, and the atraumatic ring 300 is less sharp. Thus, during implantation of the blood pump 10 into a patient's body, the peripheral edge of the liquid outlet 102 of the pump body 11 comes into contact with the inner tissue wall via the atraumatic wall 310 of the atraumatic ring 300. Since the atraumatic wall 310 exhibits a pronounced atraumatic property, the atraumatic wall 310 is less likely to scratch the inner tissue wall, thereby reducing the risk of the peripheral edge of the liquid outlet 102 causing damage to the inner tissue wall.

The pump casing 100 includes a main body section 110 and a variable diameter section 120 connected to the main body section 110. The main body section 110 is provided with the liquid inlet 101. The atraumatic ring 300 is disposed around a peripheral edge of a distal end of the variable diameter section 120, such that an inner circumference of the atraumatic ring 300 and an inner circumference of the variable diameter section 120 together define the liquid outlet 102. A direction from the impeller 220 to the liquid outlet 102 is defined as a first direction. The diameter of the liquid outlet 102 is configured to gradually increase in the first direction, such that the liquid outlet 102 has a flared shape. This configuration ensures that the atraumatic ring 300 does not block the flow surface of the liquid outlet 102. The area of the flow surface of the liquid outlet 102 gradually increases from the proximal end to a distal end of the liquid outlet 102, so that the resistance exerted by the liquid outlet 102 on blood flow is reduced, allowing blood to be discharged quickly through the liquid outlet 102, thereby effectively increasing the volume of blood discharged through the liquid outlet 102.

Specifically, the pump casing 100 and the impeller 220 are coaxial, and both have a central axis *M*1*M*2. The central axis *M*1*M*2 is an imaginary line passing through the center of the cross-section of the pump casing 100 and extending in the length direction of the pump casing 100, with *M*1 and *M*2 being two points on the imaginary line. The direction from *M*1 to *M*2 represents the direction from the proximal end to the distal end of the blood pump 10. The first direction is the direction along the central axis *M*1*M*2 from *M*1 to *M*2. It may be understood that, since the inner circumference of the atraumatic ring 300 and the inner circumference of the variable diameter section 120 together define the liquid outlet 102, setting the diameter of the liquid outlet 102 to gradually increase in the first direction means setting both the inner diameter of the atraumatic ring 300 and the inner diameter of the variable diameter section 120 to gradually increase in the first direction.

In the technical solution of the disclosure, the variable diameter section 120 is disposed on the pump casing 100, and the atraumatic ring 300 is disposed around the peripheral edge of the distal end of the variable diameter section 120, such that the inner circumference of the atraumatic ring 300 and the inner circumference of the variable diameter section 120 together define the liquid outlet 102. The diameter of the liquid outlet 102 gradually increases in the direction from the impeller 220 to the liquid outlet 102, such that the liquid outlet 102 has a flared shape. This configuration ensures that the atraumatic ring 300 does not block the flow surface of the liquid outlet 102. The area of the flow surface of the liquid outlet 102 gradually increases from the proximal end to the distal end of the liquid outlet 102, so that the resistance exerted by the liquid outlet 102 on blood flow is reduced, allowing blood to be discharged quickly through the liquid outlet 102, thereby effectively increasing the volume of blood discharged through the liquid outlet 102. Furthermore, the atraumatic ring 300 is provided with the atraumatic wall 310. The atraumatic wall 310 extends in the circumferential direction of the atraumatic ring 300, and the radial width *H*1 of the atraumatic wall 310 in a radial direction of the pump casing 100 is greater than the wall thickness *H*2 of the pump casing 100. This ensures that the radial width *H*1 of the atraumatic wall 310 is relatively large, so that the surface area of the atraumatic wall 310 is increased and the atraumatic property of the atraumatic ring 300 is enhanced, and the atraumatic ring 300 is less sharp. Thus, during implantation of the blood pump 10 into a patient's body, the peripheral edge of the liquid outlet 102 of the pump body 11 comes into contact with the inner tissue wall via the atraumatic wall 310 of the atraumatic ring 300. Since the atraumatic wall 310 exhibits a pronounced atraumatic property, the atraumatic wall 310 is less likely to scratch the inner tissue wall, thereby reducing the risk of the peripheral edge of the liquid outlet 102 causing damage to the inner tissue wall, and improving the safety of implanting the blood pump 10 into the patient's body.

Referring to FIGS. 15 - 17, for the pump casing 100 of the pump body 11, the main body section 110 of the pump casing 100 may be configured as a straight tube shape or other non-straight tube shapes. In this embodiment, the main body section 110 of the pump casing 100 is configured as a straight tube shape. The inner peripheral surface of the main body section 110 is defined as a first inner peripheral surface 111, and the outer peripheral surface of the main body section 110 is defined as a first outer peripheral surface 112. Both the first inner peripheral surface 111 and the first outer peripheral surface 112 are cylindrical surfaces. For the variable diameter section 120 of the pump casing 100, the variable diameter section 120 may be integrally formed with the atraumatic ring 300. The inner peripheral surface of the variable diameter section 120 is defined as a second inner peripheral surface 121, and the outer peripheral surface of the variable diameter section 120 is defined as a second outer peripheral surface 122. The second inner peripheral surface 121 is connected between the first inner peripheral surface 111 of the main body section 110 and the atraumatic wall 310, and constitutes a proximal portion of the inner surface of the liquid outlet 102. The second outer peripheral surface 122 is connected between the first outer peripheral surface 112 of the main body section 110 and the atraumatic wall 310.

Optionally, the inner peripheral surface of the variable diameter section 120 (i.e., the second inner peripheral surface 121) has a first diameter *C*1, and the first diameter *C*1 constitutes an inner diameter of the variable diameter section 120. The first diameter *C*1 gradually increases in the first direction, such that the liquid outlet 102 gradually increases in the first direction. The outer peripheral surface of the variable diameter section 120 (i.e., the second outer peripheral surface 122) has a second diameter *C*2, and the second diameter *C*2 may remain constant in the first direction, such that the second outer peripheral surface 122 is cylindrical. The wall thickness of the pump casing 100 at the variable diameter section 120 is defined as a first wall thickness *H*2a. The first wall thickness *H*2a is half of a difference between the second diameter *C*2 and the first diameter *C*1, i.e., *H*2a = (*C*2 - *C*1)/2, and *H*2a < *H*1.

If a difference between the radial width *H*1 of the atraumatic wall 310 and the first wall thickness *H*2a is defined as a transition step difference Δ*H*, then Δ*H* = *H*1 - *H*2a = *H*1 - (*C*2 - *C*1)/2. It may be understood that, the larger the transition step difference Δ*H*, the more likely a sharp and abrupt transition step may be formed at the junction between the variable diameter section 120 and the atraumatic wall 310. Therefore, on the basis that the first diameter *C*1 of the variable diameter section 120 gradually increases in the first direction, if the second diameter *C*2 of the variable diameter section 120 remains constant in the first direction, the first wall thickness *H*2a may gradually decrease in the first direction, and the transition step difference Δ*H* may gradually increase in the first direction. Consequently, the transition step difference Δ*H* may reach the maximum near the junction between the variable diameter section 120 and the atraumatic wall 310, so that a sharp transition step is more likely to form at the junction between the variable diameter section 120 and the atraumatic wall 310.

In view of the above, in this embodiment, the second diameter *C*2 of the variable diameter section 120 is set to gradually increase in the first direction. This configuration allows the variable diameter section 120 to assume an outwardly flared shape relative to the main body section 110. The wall thickness *H*2a of the variable diameter section 120 gradually increases, remains constant, or decreases to a lesser extent in the first direction. All of these configurations enable the transition step difference Δ*H* to be relatively small near the junction between the variable diameter section 120 and the atraumatic wall 310, so that the distal end of the second outer peripheral surface 122 and the outer peripheral edge of the atraumatic wall 310 can be relatively smoothly connected, thereby avoiding the formation of a sharp transition step at the junction between the two.

Further, the first wall thickness *H*2a of the variable diameter section 120 gradually increases in the first direction, i.e., the difference between the second diameter *C*2 and the first diameter *C*1 gradually increases in the first direction. Obviously, as the diameter of the liquid outlet 102 gradually increases in the first direction, the first wall thickness *H*2a of the variable diameter section 120 gradually increases, rather than decreasing, in the first direction. As a result, the first wall thickness *H*2a at the distal end of the variable diameter section 120 is relatively large, which can greatly reduce the transition step difference Δ*H* at the junction between the variable diameter section 120 and the atraumatic wall 310. Accordingly, the variable diameter section 120 and the atraumatic wall 310 can be connected more smoothly, thereby reducing a likelihood that a relatively sharp transition step is formed at the junction between the variable diameter section 120 and the atraumatic wall 310.

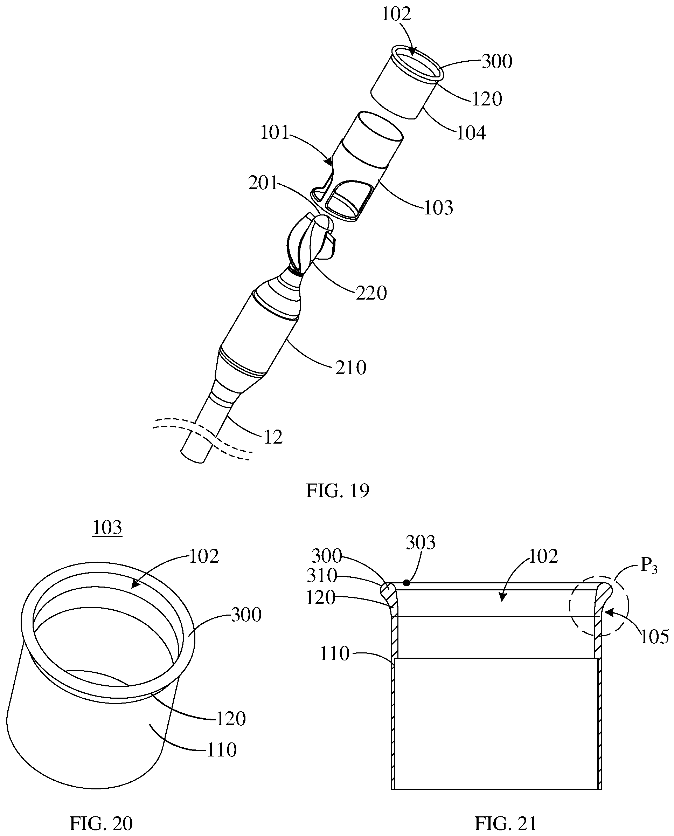

Referring to FIGS. 20 - 22, specifically, the second inner peripheral surface 121 is curved in an arc shape to be smoothly connected to the inner peripheral edge of the atraumatic wall 310. The smooth connection means that no sharp edges are formed at the junction. For example, the junction is configured as a rounded corner or an arc shape to achieve the smooth connection. In this way, the second inner peripheral surface 121 smoothly transitions to the atraumatic wall 310, so that the inner surface of the liquid outlet 102 is relatively smooth, and resistance to blood flow is reduced, and thus blood can be discharged more rapidly through the liquid outlet 102 and blood output is increased. However, the second outer peripheral surface 122 may be curved in an arc shape to smoothly connect to the outer peripheral edge of the atraumatic wall 310. In this way, the outer surface of the distal end of the pump casing 100 becomes smoother. Consequently, when the distal end of the pump casing 100 abuts against the inner tissue wall during implantation of the blood pump 10, it is less likely to scratch the inner tissue wall, thereby improving the safety of implanting the blood pump 10 into a patient's body. Optionally, both the second inner peripheral surface 121 and the second outer peripheral surface 122 are curved in a circular arc shape.

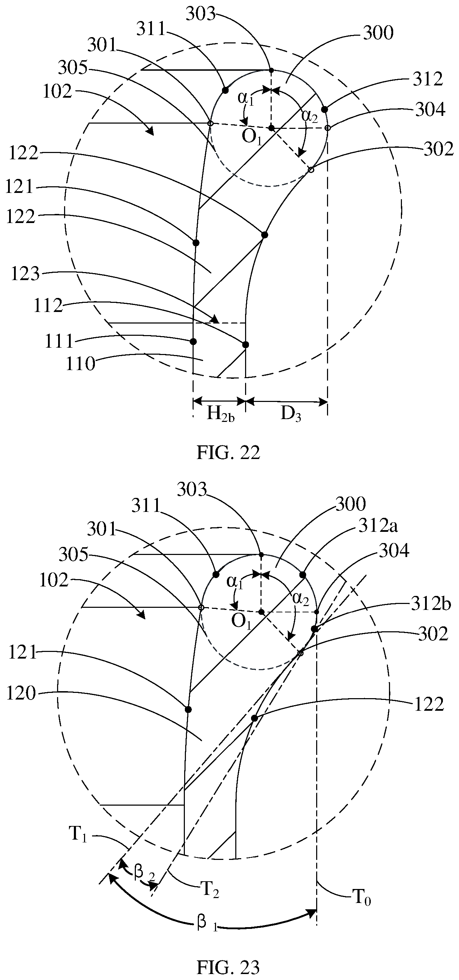

Referring to FIG. 21 and FIG. 22, further, a curvature of the second outer peripheral surface 122 is greater than a curvature of the second inner peripheral surface 121. Assuming that the radius of the arc where the second inner peripheral surface 121 is located is *R*1, and the radius of the arc where the second outer peripheral surface 122 is located is *R*2, then 1/*R*2 > 1/*R*1, i.e., *R*1 > *R*2. With such an arrangement, the curvature of the second outer peripheral surface 122 is greater than the curvature of the second inner peripheral surface 121, so that the first wall thickness *H*2a of the variable diameter section 120 gradually increases in the first direction, thereby effectively reducing the transition step difference Δ*H* and thus ensuring that the variable diameter section 104 can be better integrated with the atraumatic ring 300.

Referring to FIG. 21 and FIG. 22, for the atraumatic wall 310, the inner peripheral edge of the atraumatic wall 310 is smoothly connected to the inner peripheral surface of the variable diameter section 120. The outer peripheral edge of the atraumatic wall 310 is smoothly connected to the outer peripheral surface of the variable diameter section 120. The smooth connection means that no sharp edges are formed at the junction. For example, the junction is configured as a rounded corner or an arc shape to achieve the smooth connection. Specifically, the inner peripheral edge of the atraumatic wall 310 is connected to the inner peripheral surface of the variable diameter section 120 (i.e., the second inner peripheral surface 121), forming a first junction point 301. The outer peripheral edge of the atraumatic wall 310 is connected to the outer peripheral surface of the variable diameter section 120 (i.e., the second outer peripheral surface 122), forming a second junction point 302. The inner peripheral edge of the atraumatic wall 310 being smoothly connected to the inner peripheral surface of the variable diameter section 120 means that no sharp edges are formed at the first junction point 301, so that the inner surface of the liquid outlet 102 is relatively smooth, thereby reducing blood flow resistance. Similarly, the outer peripheral edge of the atraumatic wall 310 being smoothly connected to the outer peripheral surface of the variable diameter section 120 means that no sharp edges are formed at the second junction point 302, thereby preventing the second junction point 302 from scratching the inner tissue wall.

The atraumatic wall 310 is curved in a convex arc shape from the first junction point 301 to the second junction point 302. This configuration allows the atraumatic wall 310 to be generally rounded and smooth. Moreover, the inner peripheral edge of the atraumatic wall 310 can transition smoothly via its own arc shape to the inner peripheral surface of the variable diameter section 120, thereby achieving a smooth connection with the inner peripheral surface of the transition section 120. Similarly, the outer peripheral edge of the atraumatic wall 310 can transition smoothly via its own arc shape to the outer peripheral surface of the variable diameter section 120, thereby achieving a smooth connection with the outer peripheral surface of the variable diameter section 120. In this way, the peripheral edge of the liquid outlet 102 can be prevented from being excessively sharp, so that the liquid outlet 102 is less likely to scratch the inner tissue wall, thereby avoiding scratching of the patient's internal tissue by the distal end of the pump body 11 during implantation of the blood pump 10. Specifically, the atraumatic wall 310 is a convex arc protruding in a direction away from the main body section 110. The atraumatic wall 310 is curved in a convex arc, and the convex arc in which the atraumatic wall 310 located may specifically be a circular arc or an elliptical arc. However, the shape of the atraumatic wall 310 is not limited thereto. In other embodiments, the atraumatic wall 310 may be configured as a flat wall extending in the radial direction of the pump casing 100. Further, the inner edge of the atraumatic wall 310 is provided with a first fillet, and is smoothly connected to the inner peripheral surface of the variable diameter section 120 (i.e., the second inner peripheral surface 121) via the first fillet. The outer peripheral edge of the atraumatic wall 310 is provided with a second fillet, and is smoothly connected to the outer peripheral surface of the variable diameter section 120 (i.e., the second outer peripheral surface 122) via the second fillet. Consequently, the atraumatic wall 310 can exhibit a pronounced atraumatic property.

Referring to FIGS. 16 - 18, the first outer peripheral surface 112 of the main body section 110 has a third diameter *C*3. The atraumatic wall 310 has a radial section *S*1 perpendicular to the central axis *M*1*M*2 of the impeller 220. A circular line of tangency between the atraumatic wall 310 and the radial section S1 is defined as a distal circumferential line 303. The distal circumferential line 303 is located between the first junction point 301 and the second junction point 302. The distal circumferential line 303 has an inner circle diameter *E*. The inner circle diameter *E* is greater than the third diameter *C*3 of the main body section 110, i.e., *E* > *C*3. It may be understood that, since the distal circumferential line 303 is the circular line of tangency between the atraumatic wall 310 and the radial section *S*1, the axial distance between the distal circumferential line 303 and the main body section 110 is greater than the axial distance between any other circumferential line of the atraumatic wall 310 and the main body section 110.

Specifically, since the atraumatic ring 300 is disposed on the distal end of the variable diameter section 120, and the inner circumferential space of the atraumatic ring 300 is a part of the liquid outlet 102, the inner circle diameter *E* of the atraumatic wall 310 corresponds to the maximum diameter of the liquid outlet 102. By setting the inner circle diameter *E* to be greater than the third diameter *C*3, the maximum diameter of the liquid outlet 102 may be larger than the third diameter *C*3 of the main body section 110, so that the area of the flow surface of the liquid outlet 102 is larger than the area of the cross-section of the main body section 110. In this way, the area of the flow surface of the liquid outlet 102 is greatly increased, thereby increasing the blood output of the liquid outlet 102.

Referring to FIGS. 15 - 17, it may be understood that, the variable diameter section 120 extends a certain distance from a distal end of the main body section 110 in a direction away from the main body section 110, so that the inner diameter of the variable diameter section 120 gradually increases in the direction from the impeller 220 to the liquid outlet 102, resulting in that the liquid outlet 102 has a flared shape. Therefore, the variable diameter section 120 has a starting end 123 connected to the main body section 110 and a distal end away from the main body section 110. The starting end 123 corresponds to the minimum inner diameter position of the liquid outlet 102, and the distal end is connected to the atraumatic ring 300.

Referring to FIGS. 16 - 18, a radial distance between the distal circumferential line 303 of the atraumatic wall 310 and the first inner peripheral surface 111 of the main body section 110 is defined as an expansion deviation *D*1. An axial distance between the starting end 123 of the variable diameter section 120 and the radial section *S*1 is defined as a total outlet length *D*2. The total outlet length *D*2 is greater than the expansion deviation *D*1. It may be understood that, the expansion deviation *D*1 is equal to half of the difference between the minimum diameter and the maximum diameter of the liquid outlet 102. The total outlet length *D*2 is equal to the total axial length of the liquid outlet 102. By setting the total outlet length *D*2 to be greater than the expansion deviation *D*1, it may be ensured that the total axial length of the liquid outlet 102 is relatively long. When blood is discharged through the liquid outlet 102, the blood can be guided by the inner surface of the liquid outlet 102 to flow for a certain distance, thereby enhancing the flow-guiding effect of the liquid outlet 102 for blood discharge.

Optionally, a ratio of the total outlet length *D*2 to the expansion deviation *D*1 ranges from 2.5 to 4.5, i.e., 2.5 ≤ *D*2/*D*1 ≤ 4.5. By limiting 2.5 ≤ *D*2/*D*1 ≤ 4.5, it can be ensured that the total outlet length *D*2 can reach 2.5 to 4.5 times the expansion deviation *D*1, so that the total outlet length of the liquid outlet 102 is relatively large, which is beneficial for the liquid outlet 102 to guide blood to diffuse and discharge. On the other hand, assuming that an inclination angle at which the second inner peripheral surface 121 of the variable diameter section 120 is inclined outward relative to the first inner peripheral surface 111 of the main body section 110 is *θ*, then *θ* ≤ arctan (*D*1/*D*2). In this embodiment, since *D*2/*D*1 satisfies 2.5 ≤ *D*2/*D*1 ≤ 4.5, a maximum value of *D*1/*D*2 may be defined accordingly, thereby obtaining a suitable maximum value of the inclination angle *θ*. In this way, the inclination angle *θ* is prevented from being excessively large, and the height by which the variable diameter section 120 protrudes radially outward relative to the first outer peripheral surface 112 of the main body section 110 is prevented from being excessively large, thereby reducing the difficulty in passing the variable diameter section 120 through blood vessels in a patient's body.

Referring to FIGS. 15 - 17, a plane perpendicular to the central axis *M*1*M*2 of the impeller 220 and passing through the starting end 123 is defined as a radial reference plane *S*2. The impeller 220 is disposed inside the main body section 110, and the distal end of the impeller 220 extends to the radial reference plane *S*2, or the distal end of the impeller 220 passes through the radial reference plane *S*2 to extend into the variable diameter section 120. Specifically, the impeller 220 includes a hub 221 and a blade 222 disposed on the hub 221. The distal end of the hub 221 extends to the radial reference plane *S*2 or passes through the radial reference plane *S*2. The radial reference plane *S*2 is parallel to and spaced apart from the radial section *S*1. If the distal end of the hub 221 extends to the radial reference plane *S*2, the distal end of the impeller 220 can extend flush with the starting end 123 of the variable diameter section 120, and both are located on the radial reference plane *S*2. Particularly, if the distal end of the hub 221 passes through the radial reference plane *S*2, the distal end of the impeller 220 can extend into the interior of the variable diameter section 120, i.e., into the liquid outlet 102.

During operation of the blood pump 10, the impeller 220 rotates and drives the blood in the main body section 110 to spirally advance toward the variable diameter section 120. When the blood separates from the impeller 220, a portion of the blood *F*1 has axial potential energy to flow in the axial direction of the impeller 220, and this portion of blood is directly discharged axially through the central region of the liquid outlet 102. Another portion of the blood *F*2 and *F*3 has radial potential energy to deflect in the radial direction of the impeller 220, and this portion of blood undergoes radial deflection at the starting end of the variable diameter section 120 and come into contact with the second inner peripheral surface 121 of the variable diameter section 120. Later, this portion of blood is guided by the second inner peripheral surface 121 to discharge in the circumferential direction of the variable diameter section 120, so that this portion of blood is rapidly diffused and discharged circumferentially through the liquid outlet 102, thereby effectively increasing the blood output of the liquid outlet 102.

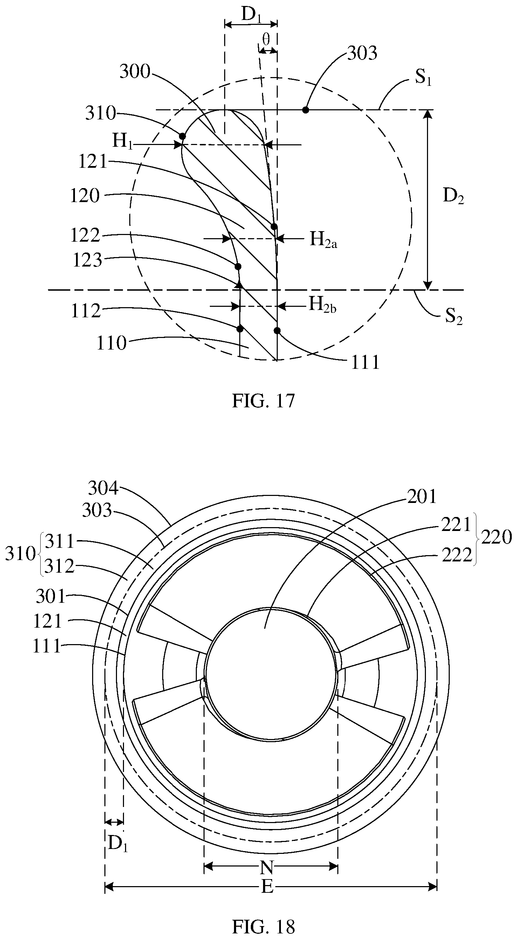

Referring to FIG. 18 and FIGS. 20 - 22, for the atraumatic ring 300, the atraumatic wall 310 can make the surface of the atraumatic ring 300 more rounded and smooth, thereby reducing the likelihood of scratching the inner tissue wall. Optionally, the atraumatic wall 310 is located on a torus, so that the atraumatic wall 310 is curved in a circular arc shape from the first junction point 301 to the second junction point 302. A center *O*1 of a generating circle 305 of the torus is positioned inside the atraumatic ring 300, and the generating circle 305 has a diameter equal to the radial width *H*1 of the atraumatic ring 300. The torus refers to a ring-shaped structure formed by rotating a circle around an axis that does not intersect the circle. The circle is referred to as the generating circle 305 of the torus. For example, structures such as swimming rings, doughnuts, and hula hoops all have toroidal shapes.

Specifically, the atraumatic ring 300 may be entirely configured as a torus, so that the entire surface of the atraumatic ring 300 forms the atraumatic wall 310. In this case, the radial width *H*1 of the atraumatic wall 310 is the diameter of the generating circle 305 of the torus. Alternatively, the atraumatic ring 300 may not be a torus, but only a portion of the surface of the atraumatic ring 300 is configured as the atraumatic wall 310. The torus in which the atraumatic wall 310 is located is a virtual torus used to design the shape of the atraumatic wall 310. Regardless of whether the torus in which the atraumatic wall 310 is located is the atraumatic ring 300 or a virtual torus, the center *O*1 of the generating circle 305 of the torus is positioned inside the atraumatic ring 300, so that the atraumatic wall 310 protrudes outwardly relative to the interior of the atraumatic ring 300, thereby making the periphery of the atraumatic ring 300 relatively rounded and smooth. When the blood pump 10 is implanted into a patient's body, the distal end of the pump body 11 comes into contact with the inner tissue wall via the atraumatic ring 300. Since the atraumatic wall 310 of the atraumatic ring 300 is relatively rounded and smooth, the inner tissue wall is less likely to be scratched, thereby avoiding damage to the patient's internal tissue.

Referring to FIGS. 21 - 23, in an embodiment, the circular line of tangency between the atraumatic wall 310 and the radial section *S*1 is defined as the distal circumferential line 303. Taking the distal circumferential line 303 as a boundary, the distal circumferential line 303 divides the atraumatic wall 310 into an inner arcuate wall 311 and an outer arcuate wall 312. The inner arcuate wall 311 is located between the first junction point 301 and the distal circumferential line 303, and constitutes the distal portion of the inner surface of the liquid outlet 102. The outer arcuate wall 312 is located between the distal circumferential line 303 and the second junction point 302. The outer arcuate wall 312 is connected to the second outer peripheral surface 122 to form the second junction point 302. Optionally, the inner arcuate wall 311 has a first central angle α1, and the first central angle α1 is less than or equal to 90°, i.e., α1 ≤ 90°. The outer arcuate wall 312 has a second central angle *α*2, and the second central angle *α*2 is greater than 90°, i.e., *α*2 > 90°.

Regarding the first central angle *α*1, it is evident that *α*1 > 0°. If *α*1 > 90°, the first junction point 301 between the inner arcuate wall 311 and the second inner peripheral surface 121 may be recessed toward the outer arcuate wall 312, and such a recess is likely to obstruct blood flow and increase resistance to blood discharge. Therefore, in this embodiment, by setting *α*1 ≤ 90°, the first junction point 301 can be made relatively smooth, so that the inner arcuate wall 311 and the second inner peripheral surface 121 are smoothly connected to form a continuous smooth inner surface. Moreover, the smooth inner surface is inwardly convex, which is beneficial for reducing resistance to blood discharge, thereby allowing blood to flow out more smoothly through the liquid outlet 102.

Regarding the second central angle *α*2, the second central angle *α*2 theoretically satisfies 0° < *α*2 ≤ 180°. However, considering that the radial width *H*1 of the atraumatic wall 310 is greater than the wall thickness *H*2 of the pump casing 100, the atraumatic wall 310 has a highest point 304 protruding radially relative to the first outer peripheral surface 112 of the main body section 110. When *α*2 ≤ 90°, the entire outer arcuate wall 312 is located between the distal circumferential line 303 and the highest point 304, and the second junction point 302 is exactly at the highest point 304, i.e., the second junction point 302 coincides with the highest point 304. In this case, it becomes difficult to achieve a smooth connection between the outer arcuate wall 312 and the second outer peripheral surface 122, so that a protruding edge or sharp corner is likely to appear at the second junction point 302. For ease of understanding, a tangent line of the outer arcuate wall 312 at the highest point 304 is defined as an original tangent line *T*0, and a tangent line at the distal end of the second outer peripheral surface 122 is defined as a first tangent line *T*1. In this case, the original tangent line *T*0 and the first tangent line *T*1 intersect to form a first included angle *β*1. Since the first included angle *β*1 is relatively large, it is difficult for the outer arcuate wall 312 and the second outer peripheral surface 122 to be smoothly connected, and a protruding edge or sharp corner is highly likely to appear at the second junction point 302, thereby increasing the difficulty of implanting the blood pump 10 into a patient's body.

In view of the above, in this embodiment, by setting *α*2 > 90°, the highest point 304 of the atraumatic ring 300 divides the outer arcuate wall 312 into a first arcuate wall 312a and a second arcuate wall 312b. The first arcuate wall 312a is located between the distal circumferential line 303 and the highest point 304, and the second arcuate wall 312b is located between the highest point 304 and the second outer peripheral surface 122. A tangent line of the second arcuate wall 312b at the second junction point 302 is defined as a second tangent line *T*2. The second tangent line *T*2 and the first tangent line *T*1 intersect to form a second included angle *β*2, which is smaller than the first included angle *β*1, i.e., *β*2 < *β*1. In this way, the second arcuate wall 312b can be smoothly connected to the second outer peripheral surface 122 at the second junction point 302, so that the outer arcuate wall 312 and the second outer peripheral surface 122 are smoothly connected to form a relatively smooth outer surface. Moreover, as *α*2 increases, the second included angle *β*2 between the second tangent line *T*2 and the first tangent line *T*1 gradually decreases. As the second included angle *β*2 decreases to 0, the second tangent line *T*2 coincides with the first tangent line *T*1, and the optimal smoothness of the connection between the outer arcuate wall 312 and the second outer peripheral surface 122 is achieved. Consequently, protruding edges or sharp corners may be avoided at the second junction point 302, thereby preventing the second junction point 302 from scratching the inner tissue wall, which is beneficial for reducing the difficulty of implanting the blood pump 10 into a patient's body.

Referring to FIG. 15, FIG. 21, and FIG. 22, in an embodiment, a height by which the atraumatic ring 300 protrudes radially relative to the outer peripheral surface (the first outer peripheral surface 112) of the main body section 110 is defined as a radial protrusion height *D*3. The radial protrusion height *D*3 is equal to the radial distance from the highest point 304 to the first outer peripheral surface 112 of the main body section 110. It may be understood that, a larger radial protrusion height *D*3 corresponds to a pronounced atraumatic property of the atraumatic ring 300, making the atraumatic ring 300 less likely to scratch the inner tissue wall. However, a larger radial protrusion height *D*3 corresponds to a larger radial dimension of the atraumatic ring 300, which correspondingly increases the radial dimension of the distal end of the pump body 11, thereby increasing the difficulty of implanting the pump body 11 into a patient's body.

In view of this, the radial protrusion height *D*3 may be preferably kept within a certain range. Optionally, a wall thickness of the pump casing 100 at the main body section 110 is defined as a second wall thickness *H*2b, and *H*2b < *H*1. A ratio of the radial protrusion height *D*3 to the second wall thickness *H*2b ranges from 1.3 to 1.8, i.e., 1.3 ≤ *D*3/*H*2b ≤ 1.8, which is equivalent to 1.3*H*2b ≤ *D*3 ≤ 1.8*H*2b. With this configuration, the radial protrusion height *D*3 of the atraumatic ring 300 is maintained within an optimal range. In this way, it can be ensured that the radial protrusion height *D*3 of the atraumatic ring 300 is not excessively small, so that the atraumatic ring 300 exhibits a pronounced atraumatic property and is less likely to scratch the inner tissue wall. Moreover, the radial protrusion height *D*3 is not excessively large, so that the radial dimension of the distal end of the pump body 11 is relatively small, thereby reducing the difficulty in implanting the pump body 11 into a patient's body.

Referring to FIG. 19, in an embodiment, the pump casing 100 includes a casing tube 103 fixedly connected to the driving unit 200, and an extension tube 104 connected to the distal end of the casing tube 103. The variable diameter section 120 and the atraumatic ring 300 are both disposed on the extension tube 104, and the atraumatic ring 300 constitutes the most distal end of the blood pump 10. The casing tube 103 and the extension tube 104 are two separately formed components. The atraumatic ring 300 may be integrally formed with the variable diameter section 120 on the extension tube 104 to reduce the number of components and reduce processing and assembly procedures. However, in other embodiments, the atraumatic ring 300 may be manufactured separately and then fixedly connected to the extension tube 104. Specifically, in this embodiment, the atraumatic ring 300 is integrally formed with the variable diameter section 120 on the extension tube 104.

It may be understood that, only a distal portion of the extension tube 104 may be configured as the variable diameter section 120 of the pump casing 100. Alternatively, the entire extension tube 104 may be configured as the variable diameter section 120 of the pump casing 100, i.e., the extension tube 104 gradually changes in diameter from its proximal portion to its distal portion to form the variable diameter section 120, and the atraumatic ring 300 is connected to the distal end of the variable diameter section 120. If only the distal portion of the extension tube 104 is configured as the variable diameter section 120 of the pump casing 100, then a proximal portion of the extension tube 104 is configured as the main body section 120 of the pump casing 100, or the proximal portion of the extension tube 104 and the casing tube 103 are both configured as a straight tube, and after the proximal portion of the extension tube 104 is connected to the casing tube 103, the proximal portion of the extension tube 104 and the casing tube 103 together form the main body section 120 of the pump casing 100. If the entire extension tube 104 is configured as the variable diameter section 120 of the pump casing 100, the casing tube 103 is configured as the main body section 110 of the pump casing 100.

Optionally, the casing tube 103 is configured as a straight tube. The casing tube 103 is coaxial with and has the same diameter as the proximal portion of the extension tube 104. Specifically, the casing tube 103, the extension tube 104, and the impeller 220 are all coaxial, and the impeller 220 is mainly located inside the casing tube 103, extending from the casing tube 103 into the extension tube 104.

Optionally, the extension tube 104 may be of an elastic structure, so that the extension tube 104 is elastic. Consequently, during implantation of the blood pump 10, when the atraumatic ring 300 abuts against the inner tissue wall, the elastic deformation of the extension tube 104 can buffer the force exerted by the atraumatic ring 300 on the inner tissue wall. It may be understood that, since the atraumatic ring 300 and the extension tube 120 are integrally formed, both the atraumatic ring 300 and the variable diameter section 120 are elastic. The elastic structure refers to a structure supported by an elastic material, and the elastic material may be a TPU material. However, in other embodiments, the extension tube 104 may be configured as a rigid component, so that the extension tube 104 exhibits rigidity. For example, the rigid component is made of metal material.

Since the curvature of the second outer peripheral surface 122 is greater than the curvature of the second inner peripheral surface 121, an annular recess 105 is formed on the outer side of the second outer peripheral surface 122. In the case where the atraumatic ring 300 is of an elastic structure, the presence of the annular recess 105 can enhance the amplitude of elastic deformation of the atraumatic ring 300 in the axial direction, thereby further buffering the reaction force exerted by the atraumatic ring 300 on the inner tissue wall. After the blood pump 10 is inserted into the pulmonary artery 26, when the distal end of the pump body 11 is subjected to a force in the axial direction of the impeller 220, the extension tube 104 is more likely to bend and deform outward from the annular recess 105, rather than bending and deforming inward toward the inner side of the liquid outlet 102. Accordingly, the bent portion is less likely to block the liquid outlet 102.

Referring to FIG. 15 and FIG. 16, in an embodiment, the impeller 220 includes a hub 221 and a blade 222 disposed on the hub 221. A distal end of the hub 221 faces the liquid outlet 102. As introduced above, the delivery path for implanting the blood pump 10 into the pulmonary artery 26 generally starts from the inferior vena cava 20 or the superior vena cava 21, and sequentially passes through the right atrium 22, the tricuspid valve 23, the right ventricle 24, and the pulmonary valve 25 to reach the pulmonary artery 26, so as to assist the right ventricle 24 in pumping blood to the pulmonary artery 26. Compared with the delivery path for implanting a blood pump from the aorta into the left ventricle, the delivery path for implanting the blood pump 10 into the pulmonary artery 26 is characterized by its short path, numerous bends, and complex internal tissues. During implantation of the pump body 11 of the blood pump 10 into the pulmonary artery 26, when the pump body 11 passes through multiple narrow bends along the delivery path to the pulmonary artery 26, the peripheral edge of the liquid outlet 102 of the pump body 11 may abut against the inner tissue wall at the bends, causing the inner tissue wall to recess inward from the liquid outlet 102 and contact the distal end of the hub 221.

In view of the above, to prevent the distal end of the hub 221 from damaging the inner tissue wall, optionally, the diameter of the hub 221 gradually increases in the direction from the impeller 220 to the liquid outlet 102. This configuration can increase the atraumatic property of the distal end of the hub 221. During the implantation of the blood pump 10 into a patient's body, if the inner tissue wall invaginates into the liquid outlet 102 and comes into contact with the distal end of the hub 221, the hub 221 of the impeller 220 of the disclosure has a larger diameter at its distal end, resulting in enhanced atraumatic property of the distal end of the hub 221 and a larger contact area between the distal end of the hub 221 and the inner tissue wall. The distal end surface of the hub 221 can disperse the interaction force between the hub 221 and the inner tissue wall, so that the distal end of the hub 221 is less likely to scratch the inner tissue wall, thereby reducing the likelihood that the distal end of the hub 221 damages the inner wall of tissue.

Furthermore, since the diameter of the hub 221 gradually increases in the direction from the impeller 220 to the liquid outlet 102, the distance between the outer peripheral surface of the hub 221 and the inner peripheral surface of the pump casing 100 may gradually decrease in a diameter-expanding direction of the hub 221. The diameter-expanding direction is the direction from the impeller 220 to the liquid outlet 102. With such an arrangement, the distal end of the hub 221 has a relatively large diameter, so that a distance *K*2 between the outer peripheral surface of the distal end of the hub 221 and the inner peripheral surface of the pump casing 100 is smaller than a distance *K*1 between the outer peripheral surface of the proximal end of the hub 221 and the inner peripheral surface of the pump casing 100, i.e., *K*2 < *K*1. Blood enters the impeller 220 at the location of distance *K*1 and is driven by the impeller 220 into a spiral rotation, thereby being gradually pushed by the impeller 220 toward the location of distance *K*2. As the path from the position of distance *K*1 to the position of distance *K*2 gradually narrows, the blood is gradually compressed spirally during this process, so that the blood obtains greater potential energy by being driven spirally by the impeller 220, thereby accelerating the blood discharge through the liquid outlet 102.

Referring to FIG. 15, FIG. 16, and FIG. 17, since the diameter of the hub 221 gradually increases in the direction from the impeller 220 to the liquid outlet 102, the distal end of the hub 221 has the maximum hub diameter *N*. Optionally, the distal end surface of the hub 221 is configured as a hemispherical surface 201, and a sphere in which the hemispherical surface 201 is located has a diameter equal to the maximum hub diameter *N*. Assuming that the sphere in which the hemispherical surface 201 is located has a center *O*2 and a radius *R*, and *O*2 is located on the central axis *M*1*M*2, then *R* = *N*/2. In this way, the distal end of the hub 221 is relatively smooth. When the inner tissue wall abuts against the distal end of the hub 221, the inner tissue wall is less likely to be scratched by the distal end of the hub 221.

It may be understood that, the driving unit 200 further includes a motor 210, and the motor 210 is connected to the impeller 220 to drive the impeller 220 to rotate. The motor 210 is fixedly connected to the proximal end of the pump casing 100 to form a part of the pump body 11. When implanting the blood pump 10 into a patient's body, the motor 210 can be implanted together with the pump casing 100. In other embodiments, the motor 210 may be disposed outside the blood pump, i.e., disposed outside the patient's body, and the motor 210 is connected to the impeller 220 through a flexible shaft that sequentially passes through the catheter 12 and the pump casing 100. During the implantation of the blood pump 10, the motor 210 is not implanted into the patient's body together with the pump casing 100.

For the blood pump 10 described in any of the above embodiments, the pump body 11 of the blood pump 10 may be clamped and fixed by the pulmonary valve 25. FIG. 24 shows a schematic view of the pump body 11 of the blood pump 10 of the disclosure being clamped and fixed by the pulmonary valve 25, where the dashed arrow *F* in FIG. 24 indicates the blood flow direction. Referring to FIG. 24, the fixing position of the pump body 11 of the blood pump 10 in a patient's body generally involves passing a portion of the pump body 11 of the blood pump 10 through the pulmonary valve 25, so that the pump body 11 is clamped and fixed by the pulmonary valve 25. At this point, the liquid inlet 101 of the pump body 11 is located in the right ventricle 24, and the liquid outlet 102 of the pump body 11 is located in the pulmonary artery 26. Blood from the right ventricle 24 can enter the pump body 11 of the blood pump 10 through the liquid inlet 101, and then be discharged into the pulmonary artery 26 through the liquid outlet 102 of the pump body 11.

However, the disclosure takes into consideration that the heart alternately performs systolic and diastolic movements. When the heart contracts, the pulmonary artery 26 opens accordingly, allowing blood to flow from the right ventricle 24 into the pulmonary artery 26. At this time, the clamping force of the pulmonary valve 25 on the blood pump 10 is reduced, and the pump body 11 of the blood pump 10 tends to move into the pulmonary artery 26. Conversely, when the heart dilates, the pulmonary valve 25 closes accordingly, and the pulmonary valve 25 tends to carry the pump body 11 of the blood pump 10 to move toward the right ventricle 24. Since the delivery path to the right ventricle is characterized by its short length, numerous bends, and complex internal structures, the axial length of the pump body 11 of the blood pump 10 of the disclosure is designed to be relatively short, so that the pump body of the blood pump can navigate various narrow bends along the delivery path to the right ventricle. If the disclosure still adopts the method of clamping and fixing the pump body 11 by the pulmonary valve 25, the pump body 11 with a shorter axial length is susceptible to the opening and closing movements of the pulmonary valve 25, so that the pump body 11 is highly likely to loosen and detach from the pulmonary valve 25.

In view of this, the disclosure further provides another method of fixing the pump body 11 of the blood pump 10 in a patient's body. Specifically, referring to FIG. 25, in an embodiment, the pump body 11 can be disposed in the pulmonary artery 26, and is fixed in cooperation with the cardiac structure via the catheter 12 of the blood pump 10. The pump body 11 is not clamped by the pulmonary artery 26, so that the pump body 11 is less susceptible to the movement of the pulmonary valve 25, thereby fundamentally eliminating the possibility that the pump body 11 loosens and detaches from the pulmonary valve 25. Moreover, the pulmonary valve 25 clamps the catheter 12, and the diameter of the catheter 12 is smaller than the diameter of the pump body 11, so the reaction force on the pulmonary valve 25 is relatively small, thereby reducing damage to the pulmonary valve 25. During the operation of the blood pump 10, blood in the right ventricle 24 flows into the pulmonary artery 26 through the pulmonary valve 25, and is then sucked into the blood pump 10 through the liquid inlet 101at an end of the pulmonary artery 26 close to the pulmonary valve 25. After being accelerated by the blood pump 10, the blood is discharged through the distal tube opening 102 of the blood pump 10 to the end of the pulmonary artery 26 away from the pulmonary valve 25.

Generally, the pulmonary artery 26 is short and thick. The distal end of the pulmonary artery 26 (i.e., the end away from the right ventricle 24) branches into a left pulmonary artery 261 and a right pulmonary artery 262. When the pump body 11 is positioned in the pulmonary artery 26, the liquid outlet 102 of the pump body 11 is relatively close to the bifurcation of the pulmonary artery 26. Since the liquid outlet 102 of the pump body 11 has a flared shape, a portion of the blood discharged through the liquid outlet 102 has potential energy for radial deflection. A portion of the blood *F*2 deflects radially to the left and flow toward the left pulmonary artery 261. Another portion of the blood *F*3 deflects radially to the right and flow toward the right pulmonary artery 262. Obviously, configuring the liquid outlet 102 of the pump body 11 with a flared shape can facilitate the diversion of blood discharged through the liquid outlet 102 to the left pulmonary artery 261 and the right pulmonary artery 262, thereby reducing the collision between the blood and the inner tissue wall at the bifurcation of the pulmonary artery 26, and further reducing the kinetic energy loss of the blood discharged through the liquid outlet 102.

Referring to FIG. 25, since the pump body 11 of the blood pump 10 of the disclosure is suitable for being mounted in the pulmonary artery 26, the length of the pump body 11 needs to be set relatively small. In view of this, in this embodiment, the pump body 11 has a first length *L*1 (see also FIG. 2 or FIG. 14), and the first length *L*1 enables the pump body 11 to be entirely positioned in the pulmonary artery 26. The blood pump 10 further includes a catheter 12, and the catheter 12 is fixedly connected to the proximal end of the driving unit 200. The catheter 12 can be fixed in cooperation with the cardiac structure, so as to position the pump body 11 in the pulmonary artery 26.

Specifically, the first length *L*1 of the pump body 11 refers to the axial length of the pump body 11. The first length *L*1 enables the blood pump 10 to pass through the right atrium 22, the right ventricle 24, and the pulmonary valve 25, and be pushed into the pulmonary artery 26, thereby ensuring that the pump body 11 as a whole can be located in the pulmonary artery 26. That is, both the liquid inlet 101 and the liquid outlet 102 of the pump body 11 are located in the pulmonary artery 26, and the pump body 11 is not clamped by the pulmonary artery 26. The catheter 12 is fixed in cooperation with the cardiac structure, so that the position of the catheter 12 is fixed, and thus the pump body 11 is positioned in the pulmonary artery 26. The first length *L*1 of the pump body 11 needs to be adapted to both the delivery path to the right ventricle 24 and the physiological structure of the pulmonary artery 26, so as to achieve better implantation into the pulmonary artery 26.

It may be understood that, the pulmonary artery 26 is short and thick. The distal end of the pulmonary artery 26 (i.e., the end away from the right ventricle 24) branches into a left pulmonary artery 261 and a right pulmonary artery 262. Since the pump body 11 is positioned in the pulmonary artery 26, the first length *L*1 preferably does not exceed 30mm, i.e., *L*1 ≤ 30mm. If the first length *L*1 exceeds 30mm, it may be difficult for the pump body 11 to navigate various narrow bends along the delivery path to the right ventricle 24. Even if the pump body 11 can pass through the delivery path to the right ventricle 24, after the entire pump body 11 reaches the pulmonary artery, the distal end of the pump body 11 may abut against the bifurcation at the end of the pulmonary artery 26 due to the excessive length of the pump body 11. Therefore, the first length *L*1 is preferably not more than 30mm.

It may be understood that, the size or anatomical shape of the delivery path to the right ventricle 24 may vary slightly among different patients. For example, the size or distance of internal tissues in the delivery path to the right ventricle 24 may differ between pediatric patients and elderly patients, and the requirements for the first length *L*1 also differ accordingly. Therefore, in practical applications, the first length *L*1 may be set according to the actual situation of the patient. Optionally, the first length *L*1 ranges from 20mm to 30mm, i.e., 20mm ≤ *L* ≤ 30mm. The first length *L*1 may be, but is not limited to, 22mm, 25mm, 28mm, 30mm, etc. Specifically, it can be reasonably designed based on characteristics such as age, body type, or disease condition of different patients.

Referring to FIG. 25, for the blood pump 10 described in any of the above embodiments, the catheter 12 includes a proximal end portion 12a, a distal end portion 12b, and a shaped portion 12c. The distal end portion 12b is fixedly connected to the driving unit 200, and the shaped portion 12c is adjacent to the distal end portion 12b. The shape of the shaped portion 12c is pre-shaped to match the anatomical shape of the blood flow path of the right ventricle 24 of the heart, so as to be fixed in cooperation with the cardiac structure. The blood flow path connects the superior vena cava 21 or the inferior vena cava 20 to the right atrium 22, the right ventricle 24, and the pulmonary artery 26.

Specifically, the catheter 12 is used to accommodate supply lines of the pump body 11 of the blood pump 10, such as wires, flushing lines, etc. The catheter 12 is of an elastic structure and can undergo elastic deformation. When not subjected to external force, the shaped portion 12c of the catheter 12 can maintain a curved initial shape, and the initial shape of the shaped portion 12c matches the anatomical shape of the blood flow path of the right ventricle 24 of the heart. During the implantation of the blood pump 10 into a patient's body, when the catheter 12 passes through the blood flow path of the heart, the shaped portion 12c of the catheter 12 is subjected to the extrusion force from the inner tissue wall of the blood flow path and undergoes elastic deformation, allowing the catheter 12 to pass smoothly through the blood flow path. After the pump body 11 of the blood pump 10 fully enters a predetermined position in the pulmonary artery 26, the shaped portion 12c of the catheter 12 returns to its initial shape, thereby achieving anatomical matching and fixation in the blood flow path of the right ventricle 24 of the heart.

For ease of understanding, the communication path among the superior vena cava 21, the right atrium 22, the right ventricle 24, and the pulmonary artery 26 is defined herein as a first blood flow path. The communication path among the inferior vena cava 20, the right atrium 22, the right ventricle 24, and the pulmonary artery 26 is defined as a second blood flow path. Referring to FIG. 25, in an embodiment, the shape of the shaped portion 12c of the catheter 12 is configured to match the shape of the first blood flow path. In this case, the delivery path for the blood pump 10 is as follows: the pump body 11 of the blood pump 10 passes sequentially through the superior vena cava 21, the right atrium 22, the tricuspid valve 23, the right ventricle 24, and the pulmonary valve 25, and enters the pulmonary artery 26. After the entire pump body 11 reaches a predetermined position in the pulmonary artery 26, the shaped portion 12c of the catheter 12 returns to its initial shape, and the shaped portion 12c matches the shape of the first blood flow path and is fixed in the first blood flow path. When not subjected to external force, the shaped portion 12c of the catheter 12 is unlikely to move in the first blood flow path.

Referring to FIG. 26, in another embodiment, the shape of the shaped portion 12c of the catheter 12 may be configured to match the shape of the second blood flow path. In this case, the delivery path for the blood pump 10 is as follows: the pump body 11 of the blood pump 10 passes sequentially through the inferior vena cava 20, the right atrium 22, the tricuspid valve 23, the right ventricle 24, and the pulmonary valve 25, and enters the pulmonary artery 26. After the entire pump body 11 reaches a predetermined position in the pulmonary artery 26, the shaped portion 12c of the catheter 12 returns to its initial shape, and the shaped portion 12c matches the shape of the second blood flow path and is fixed in the second blood flow path. When not subjected to external force, the shaped portion 12c of the catheter 12 is unlikely to move in the second blood pathway.

As described above, the entire pump body 11 of the blood pump 10 can extend into the pulmonary artery 26. Since the pulmonary artery 26 is short and thick, the pump body 11 may not be excessively long. Therefore, in the disclosure, the exit cover 300 is the most distal component of the blood pump 10, i.e., no components such as a pigtail catheter are connected to the distal side of the exit cover 300. On one hand, this ensures that the pump body 11 is not excessively long overall, and after implantation, the pump body 11 can be entirely accommodated in the pulmonary artery 26, and is less likely to abut against the bifurcation of the pulmonary artery 26.

The technical features of the above-described embodiments can be combined arbitrarily, and not all possible combinations of the technical features of the above-described embodiments have been described for the sake of simplicity of illustration. However, as long as there is no contradiction between the combinations of these technical features, it may be considered the combinations falls in the scope of this specification. The above-described embodiments only illustrate several embodiments of the disclosure, and the illustration is relatively specific and detailed. However, this cannot be understood as limiting the scope of the disclosure. It may be noted that, for those of ordinary skilled in the art, a number of variations and modifications can be made without departing from the concept of the disclosure, and all of those variations and modifications fall within the scope of protection of the disclosure. Therefore, the scope of protection of the disclosure shall be subject to the appended claims.

## Claims

1. A blood pump, the blood pump comprising a pump body, and the pump body comprising:
a pump casing, wherein a proximal end of the pump casing is provided with a liquid inlet, a distal end of the pump casing is open to define the distal end of the pump casing as a liquid outlet, and a central axis of the pump casing passes through the liquid outlet;
a driving unit, wherein the driving unit is fixedly connected to the proximal end of the pump casing, and is configured to drive blood to flow from the liquid inlet to the liquid outlet; and
an atraumatic ring, wherein the atraumatic ring is disposed around a peripheral edge of the liquid outlet, a radial thickness of the atraumatic ring is greater than a wall thickness of the pump casing, the atraumatic ring is provided with an annular wall extending in a circumferential direction of the atraumatic ring, and a center of a generating circle of a torus in which the annular wall is located is positioned inside the atraumatic ring.

2. The blood pump according to claim 1, wherein the atraumatic ring constitutes a most distal end of the blood pump;
the pump body has a first length, wherein the first length ranges from 20mm to 30mm, and the first length enables the pump body to be entirely positioned inside a pulmonary artery.

3. The blood pump according to claim 1, wherein the annular wall of the atraumatic ring comprises a distal side wall located at a most distal end of the atraumatic ring, and a cross-section of the distal side wall taken along an axial plane is semicircular, wherein the axial plane is a plane passing through the central axis.

4. The blood pump according to claim 3, wherein the annular wall further comprises at least one of the following:
the annular wall further comprises an outer side wall located on an outer side of the atraumatic ring, a cross-section of the outer side wall taken along the axial plane is arc-shaped, and the outer side wall smoothly connects the distal side wall and an outer peripheral surface of the pump casing; and
the annular wall further comprises an inner side wall located on an inner side of the atraumatic ring, a cross-section of the inner side wall taken along the axial plane is arc-shaped, and the inner side wall smoothly connects the distal side wall and an inner peripheral surface of the pump casing.

5. The blood pump according to claim 3, wherein the atraumatic ring is configured as a torus, the atraumatic ring serves as the torus in which the annular wall is located, and two cross-sections of the atraumatic ring taken along any axial plane are symmetrical with respect to the central axis.

6. The blood pump according to claim 1, wherein the pump casing comprises a casing tube fixedly connected to the driving unit and comprises an extension tube connected to a distal end of the casing tube, wherein a distal end of the extension tube is provided with a distal open end that defines the liquid outlet, the atraumatic ring is connected to a peripheral edge of the distal open end, and the atraumatic ring constitutes a most distal end of the blood pump.

7. The blood pump according to claim 6, wherein the extension tube comprises a connecting section fixedly connected to the casing tube and comprises a transition section connecting the connecting section and the atraumatic ring; the extension tube further comprises at least one of the following:
the connecting section has a first inner diameter, and the atraumatic ring has a second inner diameter, wherein the second inner diameter is a minimum inner diameter of the atraumatic ring, and the second inner diameter is smaller than the first inner diameter;
the connecting section has a first outer diameter, the atraumatic ring has a second outer diameter, the second outer diameter is a maximum outer diameter of the atraumatic ring, and the second outer diameter is smaller than the first outer diameter;
the transition section has a third inner diameter, and the third inner diameter gradually decreases in a direction from the connecting section to the atraumatic ring; and
the transition section has a third outer diameter, wherein the third outer diameter first gradually decreases and then gradually increases in the direction from the connecting section to the atraumatic ring, so that an annular recess is defined on an outer side of the transition section.

8. The blood pump according to claim 6, wherein the extension tube comprises a connecting section fixedly connected to the casing tube and comprises a transition section connecting the connecting section and the atraumatic ring, wherein the transition section is provided with a plurality of through holes penetrating through a side wall of the transition section, each of the plurality of through holes has a second length extending in a circumferential direction of the extension tube, a partition wall is formed between two adjacent through holes, the partition wall has a third length extending in the circumferential direction of the extension tube, and the third length is greater than or equal to the second length.

9. The blood pump according to claim 1, wherein the driving unit comprises a motor and an impeller connected to the motor, wherein the impeller is disposed inside the pump casing and is axially opposite to the liquid outlet; the impeller comprises a hub and a blade disposed on the hub, wherein a diameter of the hub gradually increases in a direction from the motor to the impeller, such that a distal end of the hub has a fourth outer diameter, and the fourth outer diameter is a maximum diameter of the hub; and
the impeller has a fifth outer diameter, the fifth outer diameter is a maximum diameter of the impeller during rotation of the impeller as a whole; the atraumatic ring has a second inner diameter, the second inner diameter is a minimum inner diameter of the atraumatic ring, and the second inner diameter is greater than the fourth outer diameter and smaller than the fifth outer diameter.

10. The blood pump according to claim 1, wherein the driving unit comprises a motor and an impeller connected to the motor, wherein the motor and the impeller are both disposed inside the pump casing, the motor is spaced apart from an inner wall surface of the pump casing to define a blood flow passage, the blood flow passage communicates the liquid outlet with the liquid inlet, and a proximal end of the motor is fixedly connected to the proximal end of the pump casing; and
the motor comprises a motor body and a shaft support base connected to a distal end of the motor body, wherein an outer diameter of the shaft support base gradually decreases in a direction from the motor to the impeller, such that an outer peripheral surface of the shaft support base forms a conical flow-guiding surface; a plurality of rectifying plates are disposed on the conical flow-guiding surface, the plurality of rectifying plates are arranged at intervals in a circumferential direction of the conical flow-guiding surface, and the plurality of rectifying plates all extend in an axial direction of the motor.

11. A blood pump, the blood pump comprising a pump body, and the pump body comprising:
a pump casing, wherein the pump casing comprises a main body section having a liquid inlet and a variable diameter section connected to the main body section;
an atraumatic ring, wherein the atraumatic ring is disposed at a distal end of the variable diameter section, an inner circumference of the atraumatic ring and an inner circumference of the variable diameter section together define a liquid outlet, a distal end of the atraumatic ring is provided with an atraumatic wall, the atraumatic wall extends in a circumferential direction of the atraumatic ring, and a radial width of the atraumatic wall in a radial direction of the pump casing is greater than a wall thickness of the pump casing; and
a driving unit, wherein the driving unit comprises an impeller disposed inside the pump casing, the impeller faces the liquid outlet, a direction from the impeller to the liquid outlet is defined as a first direction, and a diameter of the liquid outlet gradually increases in the first direction, such that the liquid outlet has a flared shape.

12. The blood pump according to claim 11, wherein the atraumatic ring constitutes a most distal end of the blood pump, the pump body has a first length, the first length ranges from 20mm to 30mm, and the first length enables the pump body to be entirely positioned inside a pulmonary artery.

13. The blood pump according to claim 11, wherein an inner peripheral surface of the variable diameter section has a first diameter, and the first diameter gradually increases in the first direction; an outer peripheral surface of the variable diameter section has a second diameter, and the second diameter gradually increases in the first direction.

14. The blood pump according to claim 13, wherein a wall thickness at the variable diameter section is defined as a first wall thickness, the first wall thickness is half of a difference between the second diameter and the first diameter, and the first wall thickness gradually increases in the first direction; or,
the inner peripheral surface of the variable diameter section is curved in an arc shape to smoothly connect to an inner peripheral edge of the atraumatic wall, the outer peripheral surface of the variable diameter section is curved in an arc shape to smoothly connect to an outer peripheral edge of the atraumatic wall, and a curvature of the inner peripheral surface of the variable diameter section is greater than a curvature of the outer peripheral surface of the variable diameter section to define an annular recess on an outer side of the outer peripheral surface of the variable diameter section.

15. The blood pump according to claim 11, wherein the inner peripheral edge of the atraumatic wall is smoothly connected to the inner peripheral surface of the variable diameter section, forming a first junction point; the outer peripheral edge of the atraumatic wall is smoothly connected to the outer peripheral surface of the variable diameter section, forming a second junction point; the atraumatic wall is curved in a convex arc shape from the first junction point to the second junction point.

16. The blood pump according to claim 15, wherein an outer peripheral surface of the main body section has a third diameter, the atraumatic wall has a radial section perpendicular to a central axis of the impeller, a line of tangency between the atraumatic wall and the radial section is defined as a distal circumferential line, the distal circumferential line has an inner circle diameter, and the inner circle diameter is greater than the third diameter.

17. The blood pump according to claim 16, wherein a radial distance between the distal circumferential line and the inner peripheral surface of the main body section is defined as an expansion deviation; an axial distance from a starting end of the variable diameter section connected to the main body section to the radial section is defined as a total outlet length, and the total outlet length is greater than the expansion deviation; and
a ratio of the total outlet length to the expansion deviation ranges from 2.5 to 4.5.

18. The blood pump according to claim 11, wherein the atraumatic wall is located on a torus, a center of a generating circle of the torus is positioned inside the atraumatic ring, the generating circle has a diameter equal to the radial width of the atraumatic ring; a distal circumferential line of the atraumatic wall divides the atraumatic wall into an inner arcuate wall and an outer arcuate wall, the inner arcuate wall is located between the distal circumferential line and the first junction point, and the outer arcuate wall is located between the distal circumferential line and the second junction point; wherein,
the inner arcuate wall has a first central angle, and the first central angle is less than or equal to 90°; and
the outer arcuate wall has a second central angle, and the second central angle is greater than 90°.

19. The blood pump according to claim 18, wherein the atraumatic wall has a highest point protruding radially relative to the outer peripheral surface of the main body section, the highest point divides the outer arcuate wall into a first arcuate wall and a second arcuate wall, the first arcuate wall is located between the distal circumferential line and the highest point, and the second arcuate wall is located between the highest point and the outer peripheral surface of the variable diameter section; and
a distal end of the outer peripheral surface of the variable diameter section has a first tangent line, the second arcuate wall has a second tangent line at the second junction point, and the second tangent line coincides with the first tangent line.

20. The blood pump according to claim 11, wherein a wall thickness of the pump casing at the main body section is defined as a second wall thickness, a height by which the atraumatic ring protrudes radially relative to the outer peripheral surface of the main body section is defined as a radial protrusion height, and a ratio of the radial protrusion height to the second wall thickness ranges from 1.3 to 1.8; and
the pump casing comprises a casing tube fixedly connected to the driving unit and comprises an extension tube connected to a distal end of the casing tube, wherein the variable diameter section and the atraumatic ring are both disposed on the extension tube, and the extension tube is configured as an elastic structure or a rigid structure.
